# EUROPÄISCHE PATENTSCHRIFT

(11) **EP 1 181 373 B1**
(45) Veröffentlichungstag und Bekanntmachung des Hinweises auf die Patenterteilung: **26.07.2006**
(21) Anmeldenummer: 00945722.7
(22) Anmeldetag: 07.06.2000
(51) Int. Cl.: C12N 15/53, C12N 9/02, C12N 15/82, C12N 1/19, C12P 7/64, C12Q 1/68, C11C 1/00, A01H 5/00

(54) **DELTA6-ACETYLENASE UND DELTA6-DESATURASE AUS CERATODON PURPUREUS**
DELTA6-ACETYLENASE AND DELTA6-DESATURASE FROM CERATODON PURPUREUS
DELTA6-ACETYLENASE ET DELTA6-DESATURASE OBTENUES A PARTIR DE CERATODON PURPUREUS

(30) Priorität: 07.06.1999 DE 19925718; 22.12.1999 DE 19962409
(43) Veröffentlichungstag der Anmeldung: 27.02.2002
(73) Patentinhaber: BASF Plant Science GmbH, 67056 Ludwigshafen (DE)
(72) Erfinder: HEINZ, Ernst, D-22609 Hamburg (DE); STYMNE, Sten, S-269 90 Svaloev (SE); LEE, Michael, S-211 30 Malmoe (SE); GIRKE, Thomas, San Diego, CA 92128 (US); SPERLING, Petra, D-22609 Hamburg (DE); ZAEHRINGER, Ulrich, D-23845 Borstel (DE)
(86) Internationale Anmeldenummer: PCT/EP2000/005274
(87) Internationale Veröffentlichungsnummer: WO 2000/075341

(56) Entgegenhaltungen:
- WO-A-96/21022
- WO-A-97/37033
- WO-A-98/46763
- WO-A-98/46764
- GIRKE T ET AL: "IDENTIFICATION OF A NOVEL DELTA6-ACYL-GROUP DESATURASE BY TARGETED GENE DISRUPTION IN PHYSCOMITRELLA PATENS" PLANT JOURNAL,GB,BLACKWELL SCIENTIFIC PUBLICATIONS, OXFORD, Bd. 15, Nr. 1, 1998, Seiten 39-48, XP000881712 ISSN: 0960-7412 in der Anmeldung erwähnt
- KOHN GERHARD ET AL: "Biosynthesis of acetylenic fatty acids in the moss Ceratodon purpureus (Hedw.) Brid." JOURNAL OF PLANT PHYSIOLOGY, Bd. 144, Nr. 3, 1994, Seiten 265-271, XP000960337 ISSN: 0176-1617
- BEUTELMANN, PETER ET AL: "An uncommon pathway in the biosynthesis of acetylenic fatty acids in mosses" PLANT LIPID METAB., [PAP. INT. MEET. PLANT LIPIDS], 11TH (1995), MEETING DATE 1994, 546-8. EDITOR(S): KADER, JEAN-CLAUDE;MAZLIAK, PAUL. PUBLISHER: KLUWER, DORDRECHT, NETH. , XP000960428
- SPERLING PETRA ET AL: "A sphingolipid desaturase from higher plants: Identification of a new cytochrome b5 fusion protein." JOURNAL OF BIOLOGICAL CHEMISTRY, Bd. 273, Nr. 44, 30. Oktober 1998 (1998-10-30), Seiten 28590-28596, XP000882772 ISSN: 0021-9258
- SPERLING PETRA ET AL: "A bifunctional DELTA6-fatty acyl acetylenase/desaturase from the moss Ceratodon purpureus: A new member of the cytochrome b5 superfamily." EUROPEAN JOURNAL OF BIOCHEMISTRY, Bd. 267, Nr. 12, Juni 2000 (2000-06), Seiten 3801-3811, XP000960307 ISSN: 0014-2956

## Beschreibung

Die vorliegende Erfindung betrifft ein Verfahren zur Herstellung von ungesättigten Fettsäuren sowie ein Verfahren zur Herstellung von Triglyceriden mit einem erhöhten Gehalt an ungesättigten Fettsäuren. Die Erfindung betrifft weiterhin die Verwendung von DNA Sequenzen codierend für Δ6-Acetylenase/Δ6-Desaturasen bzw. Δ6-Desaturasen zur Herstellung eines transgenen nicht-humanen Organismuses bevorzugt einer transgenen Pflanze oder eines transgenen Mikroorganismus mit erhöhtem Gehalt an Fettsäuren, Ölen oder Lipiden mit Δ6-Dreifachbindungen und/oder Δ6-Doppelbindungen.

Außerdem betrifft die Erfindung eine isolierte Nukleinsäuresequenz; eine Expressionskassette enthaltend eine Nukleinesäuresequenz, einen Vektor und nicht-humane Organismen enthaltend mindestens eine Nukleinsäuresequenz bzw. eine Expressionskassette.

Fettsäuren und Triglyceride haben eine Vielzahl von Anwendungen in der Lebensmittelindustrie, der Tierernährung, der Kosmetik und im Pharmabereich. Je nachdem ob es sich um freie gesättigte oder ungesättigte Fettsäuren oder um Triglyceride mit einem erhöhten Gehalt an gesättigten oder ungesättigten Fettsäuren handelt, sind sie für die unterschiedlichsten Anwendungen geeignet, so werden beispielsweise mehrfach ungesättigte Fettsäuren Babynahrung zur Erhöhung des Nährwertes zugesetzt. Hauptsächlich werden die verschiedenen Fettsäuren und Triglyceride aus Mikroorganismen wie Mortierella oder aus Öl-produzierenden Pflanzen wie Soja, Raps, Sonnenblume und weiteren gewonnen, wobei sie in der Regel in Form ihrer Triacylglyceride anfallen. Sie können aber auch aus Tieren wie Fischen gewonnen werden. Die freien Fettsäuren werden vorteilhaft durch Verseifung hergestellt.

Je nach Anwendungszweck sind Öle mit gesättigten oder ungesättigten Fettsäuren bevorzugt, so sind z.B. in der humanen Ernährung Lipide mit ungesättigten Fettsäuren speziell mehrfach ungesättigten Fettsäuren bevorzugt, da sie einen positiven Einfluß auf den Cholesterinspiegel im Blut und damit auf die Möglichkeit einer Herzerkrankung haben. Sie finden in verschiedenen diätischen Lebensmitteln oder Medikamenten Anwendung.

Aufgrund ihrer positiven Eigenschaften hat es in der Vergangenheit nicht an Ansätzen gefehlt, Gene, die an der Synthese von Fettsäuren bzw. Triglyceriden beteiltigt sind, für die Herstellung von Ölen in verschiedenen Organismen mit geändertem Gehalt an ungesättigten Fettsäuren verfügbar zu machen. So wird in WO 91/13972 und seinem US-Äquivalent eine Δ-9-Desaturase beschrieben. In WO 93/11245 wird eine A-15-Desaturase in WO 94/11516 wird eine Δ-12-Desaturase beansprucht. Δ-6-Desaturasen werden in WO 93/06712, US 5,614,393, WO 96/21022 und WO 99/27111 beschrieben. Weitere Desaturasen werden beispielsweise in EP-A-0 550 162, WO 94/18337, WO 97/30582, WO 97/21340, WO 95/18222, EP-A-0 794 250, Stukey et al., J. Biol. Chem., 265, 1990: 20144-20149, Wada et al., Nature 347, 1990: 200-203 oder Huang et al., Lipids 34, 1999: 649-659 beschrieben. In WO 96/13591 wird eine Δ-6-Palmitoyl-ACP-Desaturase beschrieben und beansprucht. Die biochemische Charakterisierung der verschiedenen Desaturasen ist jedoch bisher nur unzureichend erfolgt, da die Enzyme als membrangebundene Proteine nur sehr schwer zu isolieren und charakterisieren sind (McKeon et al., Methods in Enzymol. 71, 1981: 12141-12147, Wang et al., Plant Physiol. Biochem., 26, 1988: 777-792).

In WO 97/37033 wird eine Δ-12-Acetylenase beschrieben. Durch dieses Enzym lassen sich ungesättigte C₁₈-Fettsäuren mit einer Dreifachbindung herstellen. Derartige Fettsäuren können neben der Anwendung in Nahrungsmittel aufgrund ihrer Reaktivität auch zur Herstellung von Polymeren verwendet werden. Sperling et al. berichtete auf einer Tagung (South Lake Tahoe, Canada, June 9 - 13, 1999) über die Klonierung eines Enzyms, das ebenfalls Dreifachbindungen in Fettsäuren einführt. Wobei sich die Substrate dieses Enzyms von denen der Δ-12-Acetylenase unterscheiden und die Dreifachbindung an anderer Position durch das Enzym in die Fettsäuren eingeführt wird.

In Hefen konnte sowohl eine Verschiebung des Fettsäurespektrums zu ungesättigten Fettsäuren hin als auch eine Steigerung der Produktivität nachgewiesen werden (siehe Huang et al., Lipids 34, 1999: 649-659, Napier et al., Biochem. J., Vol. 330, 1998: 611-614). Die Expression der verschiedenen Desaturasen in transgenen Pflanzen zeigte allerdings nicht den gewünschten Erfolg. Eine Verschiebung des Fettsäurespektrum zu ungesättigten Fettsäuren hin konnte gezeigt werden, gleichzeitig zeigte sich aber, daß die Syntheseleistung der transgenen Pflanzen stark nachließ, das heißt gegenüber den Ausgangspflanzen konnten nur geringere Mengen an Ölen isoliert werden.

Nach wie vor besteht daher ein großer Bedarf an neuen Genen, die für Enzyme kodieren, die an der Biosynthese ungesättigter Fettsäuren beteiligt sind und es ermöglichen, diese in einem technischen Maßstab herzustellen.

Es bestand daher die Aufgabe weitere Enzyme für die Synthese ungesättigter konjugierter Fettsäuren zur Verfügung zu stellen. Diese Aufgabe wurde durch eine isolierte Nukleinsäuresequenz gelöst, die für ein Polypeptid mit Δ-6-Acetylenase- und Δ-6-Desaturaseaktivität codiert, ausgewählt aus der Gruppe:
a) einer Nukleinsäuresequenz mit der in SEQ ID NO: 1 oder SEQ ID NO: 3 dargestellten Sequenz,
b) Nukleinsäuresequenzen, die sich als Ergebnis des degenerierten genetischen Codes von der in SEQ ID NO: 1 oder SEQ ID NO: 3 dargestellten Nukleinsäuresequenz ableiten,
c) Derivate der in SEQ ID NO: 1 oder SEQ ID NO: 3 dargestellten Nukleinsäuresequenz, die für Polypeptide mit der in SEQ ID NO: 2 dargestellten Aminosäuresequenzen codieren und mindestens 75 % Homologie auf Aminosäureebene aufweisen, ohne daß die enzymatische Wirkung der Polypeptide wesentlich reduziert ist.

Unter Derivate(n) sind beispielsweise funktionelle Homologe der von SEQ ID NO: 1 oder SEQ ID NO: 3 kodierten Enzyme oder deren enzymatischer Aktivität, das heißt Enzyme, die dieselben enzymatischen Reaktionen wie die von SEQ ID NO: 1 oder SEQ ID NO: 3 kodierten Enzyme katalysieren, zu verstehen. Diese Gene ermöglichen ebenfalls eine vorteilhafte Herstellung von ungesättigten Fettsäuren mit Dreifachbindungen und/oder Doppelbindungen in Δ6-Position. Unter ungesättigten Fettsäuren sind im folgenden einfach oder mehrfach ungesättigte Fettsäuren, die Dreifachbindungen und/oder Doppelbindungen aufweisen, zu verstehen. Die Dreifach- und/oder Doppelbindungen können konjugiert oder nicht konjugiert sein. Die in SEQ ID NO: 1 oder SEQ ID NO: 3 genannten Sequenzen kodieren für ein neue Enzyme, die eine Acetylenase- und/oder Δ6-Desaturase-Aktivität aufweisen.

Das erfindungsgemäße Enzym Δ⁶-Acetylenase/Δ⁶-Desaturase führt vorteilhaft in Fettsäurereste von Glycerolipiden eine cis-Doppelbindung in Position C₆-C₇ ein und/oder konvertiert eine bereits vorhandene cis-Doppelbindung in Position C₆-C₇ in eine Dreifachbindung (siehe SEQ ID NO: 1 oder SEQ ID NO: 3). Das Enzym hat außerdem eine Δ6-Desaturase-Aktivität, die vorteilhaft in Fettsäurereste von Glycerolipiden ausschließlich eine cis-Doppelbindung in Postion C₆-C₇ einführt.

Die erfindungsgemäße(n) Nukleinsäuresequenz(en) (für die Anmeldung soll der singular den plural umfassen und umgekehrt) oder Fragmente davon können vorteilhaft zur Isolierung weiterer genomischer Sequenzen über Homologiescreening verwendet werden.

Die genannten Derivate lassen sich beispielsweise aus anderen eukaryontischen Organismen wie Pflanzen wie speziell Moosen, Dinoflagellaten oder Pilze isolieren.

Weiterhin sind unter Derivaten bzw. funktionellen Derivaten der in SEQ ID NO: 1 oder SEQ ID NO: 3 genannten Sequenzen beispielsweise Allelvarianten zu verstehen, die mindestens 70 % Homologie auf der abgeleiteten Aminosäureebene, vorteilhaft mindestens 75 % Homologie, bevorzugt mindestens 80 % Homologie, besonders bevorzugt mindestens 85 % Homologie, ganz besonders bevorzugt 90 % Homologie aufweisen. Die Homologie wurde über den gesamten Aminosäurebereich berechnet. Es wurde das Programm PileUp, BESTFIT, GAP, TRANSLATE bzw. BACKTRANSLATE (= Bestandteil des Programmpaketes UWGCG, Wisconsin Package, Version 10.0-UNIX, January 1999, Genetics Computer Group, Inc., Deverux et al., Nucleic. Acid Res., 12, 1984: 387-395) verwendet (J. Mol. Evolution., 25, 351-360, 1987, Higgins et al., CABIOS, 5 1989: 151-153). Die von den genannten Nukleinsäuren abgeleitete Aminosäuresequenzen sind Sequenz SEQ ID NO: 2 oder SEQ ID NO: 4 zu entnehmen. Unter Homologie ist Identität zu verstehen, das heißt die Aminosäuresequenzen sind zu mindestens 70 % identisch. Die erfindungsgemäßen Sequenzen sind auf Nukleinsäureebene mindestens 65 % Homolog, bevorzugt mindestens 70 %, besonders bevorzugt 75 %, ganz besonders bevorzugt mindesten 80 %.

Allelvarianten umfassen insbesondere funktionelle Varianten, die durch Deletion, Insertion oder Substitution von Nukleotiden aus der in SEQ ID NO: 1 oder SEQ ID NO: 3 dargestellten Sequenz erhältlich sind, wobei die enzymatische Aktivität der abgeleiteten synthetisierten Proteine erhalten bleibt.

Solche DNA-Sequenzen lassen sich ausgehend von den in SEQ ID NO: 1 oder SEQ ID NO: 3 beschriebenen DNA-Sequenzen oder Teilen dieser Sequenzen, beispielsweise mit üblichen Hybridisierungsverfahren oder der PCR-Technik aus anderen Eukaryonten wie beispielsweise den oben genannt isolieren. Diese DNA-Sequenzen hybridisieren unter Standardbedingungen mit den genannten Sequenzen. Zur Hybrisierung werden vorteilhaft kurze Oligonukleotide beispielsweise der konservierten Bereiche, die über Vergleiche mit anderen Acetylenase- und/oder Desaturasegenen in dem Fachmann bekannter Weise ermittelt werden können, verwendet. Vorteilhaft werden die Histidin-Box-Sequenzen verwendet. Es können aber auch längere Fragmente der erfindungsgemäßen Nukleinsäuren oder die vollständigen Sequenzen für die Hybridisierung verwendet werden. Je nach der verwendeten Nukleinsäure: Oligonukleotid, längeres Fragment oder vollständige Sequenz oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca. 10°C niedriger als die von DNA:RNA-Hybriden gleicher Länge.

Unter Standardbedingungen sind beispielsweise je nach Nukleinsäure Temperaturen zwischen 42 und 58°C in einer wäßrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50 % Formamid wie beispielsweise 42°C in 5 x SSC, 50 % Formamid zu verstehen. Vorteilhafterweise liegen die Hybridisierungsbedingungen für DNA:DNA-Hybride bei 0,1 x SSC und Temperaturen zwischen etwa 20°C bis 45°C, bevorzugt zwischen etwa 30°C bis 45°C. Für DNA:RNA-Hybride liegen die Hybridisierungsbedingungen vorteilhaft bei 0,1 x SSC und Temperaturen zwischen etwa 30°C bis 55°C, bevorzugt zwischen etwa 45°C bis 55°C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Weiterhin sind unter Derivaten Homologe der Sequenz SEQ ID No: 1 oder SEQ ID NO: 3 beispielsweise eukaryontische Homologe, verkürzte Sequenzen, Einzelstrang-DNA der codierenden und nichtcodierenden DNA-Sequenz oder RNA der codierenden und nichtcodierenden DNA-Sequenz zu verstehen.

Außerdem sind unter Homologen der Sequenzen SEQ ID NO: 1 oder SEQ ID NO: 3 Derivate wie beispielsweise Promotorvarianten zu verstehen. Diese Varianten können durch ein oder mehrere Nukleotidaustausche, durch Insertion(en) und/oder Deletion(en) verändert sein, ohne daß aber die Funktionalität bzw. Wirksamkeit der Promotoren beeinträchtigt sind. Des weiteren können die Promotoren durch Veränderung ihrer Sequenz in ihrer Wirksamkeit erhöht oder komplett durch wirksamere Promotoren auch artfremder Organismen ausgetauscht werden.

Unter Derivaten sind auch vorteilhaft Varianten zu verstehen, deren Nukleotidsequenz im Bereich -1 bis -2000 vor dem Startkodon so verändert wurden, daß die Genexpression und/oder die Proteinexpression verändert, bevorzugt erhöht wird. Weiterhin sind unter Derivaten auch Varianten zu verstehen, die am 3'-Ende verändert wurden.

Unter Derivaten sind auch die Antisense-DNAs zu verstehen, die zur Hemmung der Proteinbiosynthese der erfindungsgemäßen Proteine verwendet werden können. Diese Antisense-DNAs gehören zu den erfindungsgemäßen nichtfunktionellen Derivaten, wie Derivate, die keine enzymatische Aktivität aufweisen. Weitere dem Fachmann bekannte Methoden der Herstellung von nichtfunktionellen Derivaten sind die sogenannte Cosuppression, die Verwendung von Ribozymen und Introns. Ribozyme sind katalytische RNA-Moleküle mit Ribonukleaseaktivität, die Einzelstrang Nukleinsäuren wie mRNA, zu denen sie eine Komplementarität aufweisen, schneiden können. Dadurch können mit Hilfe dieser Ribozyme (Haselhoff and Gerlach, Nature, 334, 1988: 585-591) mRNA-Transkripte katalytisch gespalten werden und so die Translation dieser mRNA unterdrückt werden. Derartige Ribozyme können speziell auf ihre Aufgaben hin zugeschnitten werden (US 4,987,071; US 5,116,742 und Bartel et al., Science 261, 1993: 1411-1418). Mit Hilfe der Antisense-DNA können dadurch Fettsäuren, Lipide oder Öle mit einem erhöhten Anteil an gesättigten Fettsäuren hergestellt werden.

Die erfindungsgemäßen Nukleinsäuresequenzen, die für eine Δ6-Acetylenase und Δ6-Desaturase kodieren, können synthetisch hergestellt oder natürlich gewonnen sein oder eine Mischung aus synthetischen und natürlichen DNA-Bestandteilen enthalten, sowie aus verschiedenen heterologen Δ6-Acetylenase und Δ6-Desaturase-Genabschnitten verschiedener Organismen bestehen. Im allgemeinen werden synthetische Nukleotid-Sequenzen mit Codons erzeugt, die von den entsprechenden Wirtsorganismen beispielsweise Pflanzen bevorzugt werden. Dies führt in der Regel zu einer optimalen Expression der heterologen Gene. Diese von Pflanzen bevorzugten Codons können aus Codons mit der höchsten Proteinhäufigkeit bestimmt werden, die in den meisten interessanten Pflanzenspezies exprimiert werden. Ein Beispiel für Corynebacterium glutamicum ist gegeben in: Wada et al. (1992) *Nucleic Acids Res.* 20:2111-2118). Die Durchführung solcher Experimente sind mit Hilfe von Standardmethoden durchführbar und sind dem Fachmann auf dem Gebiet bekannt.

Funktionell äquivalente Sequenzen, die für das Δ6-Acetylenase - und Δ6-Desaturase-Gen kodieren, sind solche Derivate der erfindungsgemäßen Sequenzen, welche trotz abweichender Nukleotidsequenz noch die gewünschten Funktionen, das heißt die enzymatische Aktivität der Proteine besitzen. Funktionelle Äquivalente umfassen somit natürlich vorkommende Varianten der hierin beschriebenen Sequenzen sowie künstliche, z.B. durch chemische Synthese erhaltene, an den Codon-Gebrauch einer Pflanze angepaßte, künstliche Nukleotid-Sequenzen.

Außerdem sind artifizielle DNA-Sequenzen geeignet, solange sie, wie oben beschrieben, die gewünschte Eigenschaft beispielsweise der Erhöhung des Gehaltes von Δ6-Dreifachbindungen oder Δ6-Doppelbindungen in Fettsäuren, Ölen oder Lipiden in der Pflanze durch Überexpression des Δ6-Acetylenase/Δ6-Desaturase- und/oder Δ6-Deasaturase-Gens in Kulturpflanzen vermitteln. Solche artifiziellen DNA-Sequenzen können beispielsweise durch Rückübersetzung mittels Molecular Modelling konstruierter Proteine, die Δ6-Acetylenase- und Δ6-Desaturase-Aktivität aufweisen oder durch in vitro-Selektion ermittelt werden. Mögliche Techniken zur in vitro-Evolution von DNA zur Veränderung bzw. Verbesserung der DNA-Sequenzen sind beschrieben bei Patten, P.A. et al., Current Opinion in Biotechnology 8, 724-733( 1997) oder bei Moore, J.C. et al., Journal of Molecular Biology 272, 336-347 (1997). Besonders geeignet sind codierende DNA-Sequenzen, die durch Rückübersetzung einer Polypeptidsequenz gemäß der für die Wirtspflanze spezifischen Kodon-Nutzung erhalten werden. Die spezifische Kodon-Nutzung kann ein mit pflanzengenetischen Methoden vertrauter Fachmann durch Computerauswertungen anderer, bekannter Gene der zu transformierenden Pflanze leicht ermitteln.

Als weitere geeignete äquivalente Nukleinsäure-Sequenzen sind zu nennen Sequenzen, welche für Fusionsproteine kodieren, wobei Bestandteil des Fusionsproteins ein Δ6-Acetylenase- und Δ6-Desaturase-Polypeptid oder ein funktionell äquivalenter Teil davon ist. Der zweite Teil des Fusionsproteins kann z.B. ein weiteres Polypeptid mit enzymatischer Aktivität sein oder eine antigene Polypeptidsequenz mit deren Hilfe ein Nachweis auf Δ6-Acetylenase- und Δ6-Desaturase-Expression möglich ist (z.B. myctag oder his-tag). Bevorzugt handelt es sich dabei jedoch um eine regulative Proteinsequenz, wie z.B. ein Signalsequenz für das ER, das das Δ6-Acetylenase- und Δ6-Desaturase-Protein an den gewünschten Wirkort leitet.

Vorteilhaft können die Δ6-Acetylenase/Δ6-Desaturase-Gene im erfindungsgemäßen Verfahren mit weiteren Genen der Fettsäurebiosynthese kombiniert werden. Beispiele für derartige Gene sind die Acetyltransferasen, weitere Desaturasen oder Elongasen. Für die in-vivo und speziell in-vitro Synthese ist die Kombination mit z.B. NADH-Cytochrom B5 Reduktasen vorteilhaft, die Reduktionsäquivalente aufnehmen oder abgeben können.

Unter den erfindungsgemäßen Aminosäuresequenzen sind Proteine zu verstehen, die eine in den Sequenzen SEQ ID NO: 2 oder SEQ ID NO: 4 dargestellte Aminosäuresequenz oder eine daraus durch Substitution, Inversion, Insertion oder Deletion von einem oder mehreren Aminosäureresten erhältliche Sequenz enthalten, wobei die enzymatische Aktivität des in SEQ ID NO: 2 oder SEQ ID NO: 4 dargestellten Proteins erhalten bleibt bzw. nicht wesentlich reduziert wird. Unter nicht wesentlich reduziert sind alle Enzyme zu verstehen, die noch mindestens 10 %, bevorzugt 20 %, besonders bevorzugt 30 % der enzymatischen Aktivität des Ausgangsenzyms aufweisen. Dabei können beispielsweise bestimmte Aminosäuren durch solche mit ähnlichen physikochemischen Eigenschaften (Raumerfüllung, Basizität, Hydrophobizität etc.) ersetzt werden. Beispielsweise werden Argininreste gegen Lysinreste, Valinreste gegen Isoleucinreste oder Asparaginsäurereste gegen Glutaminsäurereste ausgetauscht. Es können aber auch ein oder mehrere Aminosäuren in ihrer Reihenfolge vertauscht, hinzugefügt oder entfernt werden, oder es können mehrere dieser Maßnahmen miteinander kombiniert werden.

Unter Derivaten sind auch funktionelle Äquivalente zu verstehen, die insbesondere auch natürliche oder künstliche Mutationen einer ursprünglich isolierten für eine Δ6-Acetylenase/Δ6-Desaturase codierende Sequenz beinhalten, welche weiterhin die gewünschte Funktion, das heißt deren enzymatische Aktivität nicht wesentlich reduziert ist, zeigen. Mutationen umfassen Substitutionen, Additionen, Deletionen, Vertauschungen oder Insertionen eines oder mehrerer Nukleotidreste. Somit werden beispielsweise auch solche Nukleotidsequenzen durch die vorliegende Erfindung mit umfaßt, welche man durch Modifikation der Δ6-Acetylenase/Δ6-Desaturase Nukleotidsequenz erhält. Ziel einer solchen Modifikation kann z.B. die weitere Eingrenzung der darin enthaltenen codierenden Sequenz oder z.B. auch die Einfügung weiterer Restriktionsenzym-Schnittstellen sein.

Funktionelle Äquivalente sind auch solche Varianten, deren Funktion, verglichen mit dem Ausgangsgen bzw. Genfragment, abgeschwächt (= nicht wesentlich reduziert) oder verstärkt ist (= Enzymaktivität stärker als die Aktivität des Ausgangsenzym, das heißt Aktivität ist höher als 100 %, bevorzugt höher als 110 %, besonders bevorzugt höher als 130 %).

Die Nukleinsäuresequenz kann dabei vorteilhaft beispielsweise eine DNA- oder cDNA-Sequenz sein. Zur Insertion in eine erfindungsgemäße Expressionskassette geeignete codierende Sequenzen sind beispielsweise solche, die für eine Δ6-Acetylenase/Δ6-Desaturase mit den oben beschriebenen Sequenzen kodieren und die dem Wirt die Fähigkeit zur Überproduktion von Fettsäuren, Ölen oder Lipiden mit Dreifachbindungen und/oder Doppelbindungen in Δ6-Position verleihen. Diese Sequenzen können homologen oder heterologen Ursprungs sein.

Unter der erfindungsgemäßen Expressionskassette (= Nukleinsäurekonstrukt oder -fragment) sind die in SEQ ID NO: 1 oder SEQ ID NO: 3 genannten Sequenzen, die sich als Ergebnis des genetischen Codes und/oder deren funktionellen oder nicht funktionellen Derivate zu verstehen, die mit einem oder mehreren Regulationssignalen vorteilhafterweise zur Erhöhung der Genexpression funktionell verknüpft wurden und welche die Expression der codierenden Sequenz in der Wirtszelle steuern. Diese regulatorischen Sequenzen sollen die gezielte Expression der Gene und der Proteinexpression ermöglichen. Dies kann beispielsweise je nach Wirtsorganismus bedeuten, daß das Gen erst nach Induktion exprimiert und/oder überexprimiert wird, oder daß es sofort exprimiert und/oder überexprimiert wird. Beispielsweise handelt es sich bei diesen regulatorischen Sequenzen um Sequenzen an die Induktoren oder Repressoren binden und so die Expression der Nukleinsäure regulieren. Zusätzlich zu diesen neuen Regulationssequenzen oder anstelle dieser Sequenzen kann die natürliche Regulation dieser Sequenzen vor den eigentlichen Strukturgenen noch vorhanden sein und gegebenenfalls genetisch verändert worden sein, so daß die natürliche Regulation ausgeschaltet und die Expression der Gene erhöht wurde. Das Genkonstrukt kann aber auch einfacher aufgebaut sein, das heißt es wurden keine zusätzlichen Regulationssignale vor die Nukleinsäuresequenz oder dessen Derivate inseriert und der natürliche Promotor mit seiner Regulation wurde nicht entfernt. Stattdessen wurde die natürliche Regulationssequenz so mutiert, daß keine Regulation mehr erfolgt und/oder die Genexpression gesteigert wird. Diese veränderten Promotoren können in Form von Teilsequenzen (= Promotor mit Teilen der erfindungsgemäßen Nukleinsäuresequenzen) auch allein vor das natürliche Gen zur Steigerung der Aktivität gebracht werden. Das Genkonstrukt kann außerdem vorteilhafterweise auch eine oder mehrere sogenannte "enhancer Sequenzen" funktionell verknüpft mit dem Promotor enthalten, die eine erhöhte Expression der Nukleinsäuresequenz ermöglichen. Auch am 3'-Ende der DNA-Sequenzen können zusätzliche vorteilhafte Sequenzen inseriert werden wie weitere regulatorische Elemente oder Terminatoren. Die Δ6-Acetylenase-/Δ6-Desaturase-Gene können in einer oder mehreren Kopien in der Expressionskassette (= Genkonstrukt) enthalten sein.

Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Als Promotoren in der Expressionskassette sind grundsätzlich alle Promotoren geeignet, die die Expression von Fremdgenen in Organismen vorteilhaft in Pflanzen oder Pilzen steuern können. Vorzugsweise verwendet man insbesondere ein pflanzliche Promotoren oder Promotoren, die aus einem Pflanzenvirus entstammen. Vorteilhafte Regulationssequenzen für das erfindungsgemäße Verfahren sind beispielsweise in Promotoren wie cos-, tac-, trp-, tet-, trp-tet-, lpp-, lac-, lpp-lac-, lacI^{q-,}T7-, T5-, T3-, gal-, trc-, ara-, SP6-, λ-P_{R}- oder im λ-P_{L}-Promotor enthalten, die vorteilhafterweise in gram-negativen Bakterien Anwendung finden. Weitere vorteilhafte Regulationssequenzen sind beispielsweise in den gram-positiven Promotoren amy und SP02, in den Hefe- oder Pilzpromotoren ADC1, MFα, AC, P-60, CYC1, GAPDH, TEF, rp28, ADH oder in den Pflanzenpromotoren wie CaMV/35S [Franck et al., Cell 21(1980) 285-294], SSU, OCS, lib4, STLS1, B33, nos (= Nopalin Synthase Promotor) oder im Ubiquitin-Promotor enthalten. Die Expressionskassette kann auch einen chemisch induzierbaren Promotor enthalten, durch den die Expression des exogenen Δ6-ACETYLENASE/Δ6-DESATURASE-Gens in den Organismen vorteilhaft in den Pflanzen zu einem bestimmten Zeitpunkt gesteuert werden kann. Derartige vorteilhafte Pflanzenpromotoren sind beispielsweise deer PRP1-Promotor [Ward et al., Plant.Mol. Biol.22(1993), 361-366], ein durch Benzensulfonamid-induzierbarer (EP 388186), ein durch Tetrazyklin-induzierbarer (Gatz et al., (1992) Plant J. 2,397-404), ein durch Salizylsäure induzierbarer Promotor (WO 95/19443), ein durch Abscisinsäure-induzierbarer (EP335528) bzw. ein durch Ethanol- oder Cyclohexanon-induzierbarer (WO93/21334) Promotor. Weitere Pflanzenpromotoren sind beispielsweise der Promotor der cytosolischen FBPase aus Kartoffel, der ST-LSI Promotor aus Kartoffel (Stockhaus et al., EMBO J. 8 (1989) 2445-245), der Promotor der Phosphoribosylpyrophosphat Amidotransferase aus Glycine max (siehe auch Genbank Accession Nummer U87999) oder ein Nodien-spezifischen Promotor wie in EP 249676 können vorteilhaft verwandt werden. Vorteilhaft sind insbesonders solche pflanzliche Promotoren, die die Expression in Geweben oder Pflanzenteilen/-organen sicherstellen, in denen die Fettsäurebiosynthese bzw. dessen Vorstufen stattfindet wie beispielsweise im Endosperm oder im sich entwickelnden Embryo. Insbesondere zu nennen sind vorteilhafte Promotoren, die eine samenspezifische Expression gewährleisten wie beispielsweise der USP-Promotor oder Derivate davon, der LEB4-Promotor, der Phaseolin-Promotor oder der Napin-Promotor. Der besonders vorteilhafte USP-Promotor oder dessen Derivate vermitteln in der Samenentwicklung eine sehr früh Genexpression (Baeumlein et al., Mol Gen Genet, 1991, 225 (3): 459-67). Weitere vorteilhafte samenspezifische Promotoren, die für monokotyle und dikotyle Pflanzen verwendet werden können, sind die für Dikotyle geeignete Promotoren wie der Napingen-Promotor aus Raps (US5,608,152), der Oleosin-Promotor aus Arabidopsis (WO98/45461), der Phaseolin-Promotor aus Phaseolus vulgaris (US5,504,200), der Bce4-Promotor aus Brassica (WO91/13980) oder der Leguminosen B4-Promotor (LeB4, Baeumlein et al., Plant J., 2, 2, 1992: 233 - 239) oder für Monokotyle geeignete Promotoren wie die Promotoren die Promotoren des lpt2- oder lpt1-Gens aus Gerste (WO95/15389 und WO95/23230) oder die Promotoren des Gersten Hordein-Gens, des Reis Glutelin-Gens, des Reis Oryzin-Gens, des Reis Prolamin-Gens, des Weizen Gliadin-Gens, des Weizen Glutelin-Gens, des Mais Zein-Gens, des Hafer Glutelin-Gens, des Sorghum Kasirin-Gens oder des Roggen Secalin-Gens, die in WO99/16890 beschrieben werden.

Weiterhin sind insbesondere solche Promotoren bevorzugt, die die Expression in Geweben oder Pflanzenteilen sicherstellen, in denen beispielsweise die Biosynthese von Fettsäuren, Ölen und Lipiden bzw. deren Vorstufen stattfindet. Insbesondere zu nennen sind Promotoren, die eine samenspezifische Expression gewährleisten. Zu nennen sind der Promotor des Napin-Gens aus Raps (US 5,608,152), des USP-Promotor aus Vicia faba (USP = unbekanntes Samenprotein, Baeumlein et al., Mol Gen Genet, 1991, 225 (3): 459-67), des Oleosin-Gens aus Arabidopsis (WO98/45461), des Phaseolin-Promotors (US 5,504,200) oder der Promotor des Legumin B4-Gens (LeB4; Baeumlein et al., 1992, Plant Journal, 2 (2): 233-9). Weiterhin sind zu nennen Promotoren, wie der des lpt2 oder lpt1-Gens aus Gerste (WO95/15389 und WO95/23230), die in monokotylen Pflanzen samenspezifische Expression vermitteln.

In der Expressionskassette (= Genkonstrukt, Nukleinsäurekonstrukt) können wie oben beschrieben noch weitere Gene, die in die Organismen eingebracht werden sollen, enthalten sein. Diese Gene können unter getrennter Regulation oder unter der gleichen Regulationsregion wie die Gene der Δ6-ACETYLENASE/Δ6-DESATURASE liegen. Bei diesen Genen handelt es sich beispielsweise um weitere Biosynthesegene vorteilhaft der Fettsäurebiosynthese, die eine gesteigerte Synthese ermöglichen. Beispielsweise seien die Gene für die Δ15-, Δ12-, Δ9-, Δ6-, Δ5-Desaturase, die verschiedenen Hydroxylasen, die Δ12-Acetylenase, die Acyl-ACP-Thioesterasen, β-Ketoacyl-ACP-Synthasen oder β-Ketoacyl-ACP-Reductasen genannt. Vorteilhaft werden die Desaturasegene im Nukleinsäurekonstrukt verwendet.

Prinzipiell können alle natürlichen Promotoren mit ihren Regulationssequenzen wie die oben genannten für die erfindungsgemäße Expressionskassette und das erfindungsgemäße Verfahren, wie unten beschrieben, verwendet werden. Darüber hinaus können auch synthetische Promotoren vorteilhaft verwendet werden.

Bei der Präparation einer Expressionskassette können verschiedene DNA-Fragmente manipuliert werden, um eine Nukleotid-Sequenz zu erhalten, die zweckmäßigerweise in der korrekten Richtung liest und die mit einem korrekten Leseraster ausgestattet ist. Für die Verbindung der DNA-Fragmente (= erfindungsgemäße Nukleinsäuren) miteinander können an die Fragmente Adaptoren oder Linker angesetzt werden.

Zweckmäßigerweise können die Promotor- und die Terminator-Regionen in Transkriptionsrichtung mit einem Linker oder Polylinker, der eine oder mehrere Restriktionsstellen für die Insertion dieser Sequenz enthält, versehen werden. In der Regel hat der Linker 1 bis 10, meistens 1 bis 8, vorzugsweise 2 bis 6 Restriktionsstellen. Im allgemeinen hat der Linker innerhalb der regulatorischen Bereiche eine Größe von weniger als 100 bp, häufig weniger als 60 bp, mindestens jedoch 5 bp. Der Promotor kann sowohl nativ bzw. homolog als auch fremdartig bzw. heterolog zum Wirtsorganismus beispielsweise zur Wirtspflanze sein. Die Expressionskassette beinhaltet in der 5'-3'-Transkriptionsrichtung den Promotor, eine DNA-Sequenz, die für ein Δ6-Acetylenase/Δ6-Desaturase-Gen codiert und eine Region für die transkriptionale Termination. Verschiedene Terminationsbereiche sind gegeneinander beliebig austauschbar.

Ferner können Manipulationen, die passende Restriktionsschnittstellen bereitstellen oder die überflüssige DNA oder Restriktionsschnittstellen entfernen, eingesetzt werden. Wo Insertionen, Deletionen oder Substitutionen wie z.B. Transitionen und Transversionen in Frage kommen, können in *vitro*-Mutagenese, -primerrepair-, Restriktion oder Ligation verwendet werden. Bei geeigneten Manipulationen, wie z.B. Restriktion, -chewing-back- oder Auffüllen von Überhängen für -bluntends-, können komplementäre Enden der Fragmente für die Ligation zur Verfügung gestellt werden.

Von Bedeutung für eine vorteilhafte hohe Expression kann u.a. das Anhängen des spezifischen ER-Retentionssignals SEKDEL sein (Schouten, A. et al., Plant Mol. Biol. 30 (1996), 781-792), die durchschnittliche Expressionshöhe wird damit verdreifacht bis vervierfacht. Es können auch andere Retentionssignale, die natürlicherweise bei im ER lokalisierten pflanzlichen und tierischen Proteinen vorkommen, für den Aufbau der Kassette eingesetzt werden.

Bevorzugte Polyadenylierungssignale sind pflanzliche Polyadenylierungssignale, vorzugsweise solche, die im wesentlichen T-DNA-Polyadenylierungssignale aus Agrobacterium tumefaciens, insbesondere des Gens 3 der T-DNA (Octopin Synthase) des Ti-Plasmids pTiACH5 entsprechen (Gielen et al., EMBO J.3 (1984), 835 ff) oder entsprechende funktionelle Äquivalente.

Die Herstellung einer Expressionskassette erfolgt durch Fusion eines geeigneten Promotors mit einer geeigneten Δ6-Acetylenase/Δ6-Desaturase-DNA-Sequenz sowie einem Polyadenylierungssignal nach gängigen Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley-Interscience (1987) beschrieben werden.

Bei der Präparation einer Expressionskassette können verschiedene DNA-Fragmente manipuliert werden, um eine Nukleotid-Sequenz zu erhalten, die zweckmäßigerweise in der korrekten Richtung liest und die mit einem korrekten Leseraster ausgestattet ist. Für die Verbindung der DNA-Fragmente miteinander können an die Fragmente Adaptoren oder Linker angesetzt werden.

Zweckmäßigerweise können die Promotor- und die Terminator-Regionen in Transkriptionsrichtung mit einem Linker oder Polylinker, der eine oder mehrere Restriktionsstellen für die Insertion dieser Sequenz enthält, versehen werden. In der Regel hat der Linker 1 bis 10, meistens 1 bis 8, vorzugsweise 2 bis 6 Restriktionsstellen. Im allgemeinen hat der Linker innerhalb der regulatorischen Bereiche eine Größe von weniger als 100 bp, häufig weniger als 60 bp, mindestens jedoch 5 bp. Der Promotor kann sowohl nativ bzw. homolog als auch fremdartig bzw. heterolog zur Wirtspflanze sein. Die Expressionskassette beinhaltet in der 5'-3'-Transkriptionsrichtung den Promotor, eine DNA-Sequenz die für ein D6-Acetylenase/Desaturase-Gen codiert und eine Region für die transkriptionale Termination. Verschiedene Terminationsbereiche sind gegeneinander beliebig austauschbar.

Bei der Präparation einer Expressionskassette können verschiedene DNA-Fragmente manipuliert werden, um eine Nukleotid-Sequenz zu erhalten, die zweckmäßigerweise in der korrekten Richtung liest und die mit einem korrekten Leseraster ausgestattet ist. Für die Verbindung der DNA-Fragmente miteinander können an die Fragmente Adaptoren oder Linker angesetzt werden.

Zweckmäßigerweise können die Promotor- und die Terminator-Regionen in Transkriptionsrichtung mit einem Linker oder Polylinker, der eine oder mehrere Restriktionsstellen für die Insertion dieser Sequenz enthält, versehen werden. In der Regel hat der Linker 1 bis 10, meistens 1 bis 8, vorzugsweise 2 bis 6 Restriktionsstellen. Im allgemeinen hat der Linker innerhalb der regulatorischen Bereiche eine Größe von weniger als 100 bp, häufig weniger als 60 bp, mindestens jedoch 5 bp. Der Promotor kann sowohl nativ bzw. homolog als auch fremdartig bzw. heterolog zur Wirtspflanze sein. Die Expressionskassette beinhaltet in der 5'-3'-Transkriptionsrichtung den Promotor, eine DNA-Sequenz die für ein Δ6-Acetylenase/Δ6-Desaturase-Gen codiert und eine Region für die transkriptionale Termination. Verschiedene Terminationsbereiche sind gegeneinander beliebig austauschbar.

Die DNA Sequenz codierend für eine Δ6-Acetylenase/Δ6-Desaturase aus Ceratodon purpureus beinhaltet alle Sequenzmerkmale, die notwendig sind, um eine dem Ort der Fettsäure-, Lipid- oder Ölbiosynthese korrekte Lokalisation zu erreichen. Daher sind keine weiteren Targetingsequenzen per se notwendig. Allerdings kann eine solche Lokalisation wünschenswert und vorteilhaft sein und daher künstlich verändert oder verstärkt werden, so daß auch solche Fusionskonstrukte eine bevorzugte vorteilhafte Ausführungsform der Erfindung sind.

Insbesondere bevorzugt sind Sequenzen, die ein Targeting in Plastiden gewährleisten. Unter bestimmten Umständen kann auch ein Targeting in andere Kompartimente (referiert: Kermode, Crit. Rev. Plant Sci. 15, 4 (1996), 285-423) z.B. in in die Vakuole, in das Mitochondrium, in das Endoplasmatische Retikulum (ER), Peroxisomen, Lipidkörper oder durch ein Fehlen entsprechender operativer Sequenzen ein Verbleib im Kompartiment des Entstehens, dem Zytosol, wünschenswert sein.

Vorteilhafterweise werden die erfindungsgemäßen Nukleinsäuresequenzen zusammen mit mindestens einem Reportergen in eine Expressionskassette kloniert, die in den Organismus über einen Vektor oder direkt in das Genom eingebracht wird. Dieses Reportergen sollte eine leichte Detektierbarkeit über einen Wachstums-, Fluoreszenz-, Chemo-, Biolumineszenz- oder Resistenzassay oder über eine photometrische Messung ermöglichen. Beispielhaft seien als Reportergene Antibiotika-oder Herbizidresistenzgene, Hydrolasegene, Fluoreszenzproteingene, Biolumineszenzgene, Zucker- oder Nukleotidstoffwechselgene oder Biosynthesegene wie das Ura3-Gen, das Ilv2-Gen, das Luciferasegen, das β-Galactosidasegen, das gfp-Gen, das 2-Desoxyglucose-6-phosphat-Phosphatasegen, das β-Glucuronidase-Gen, β-Lactamasegen, das Neomycinphosphotransferasegen, das Hygromycinphosphotransferasegen oder das BASTA (= Gluphosinatresistenz)-Gen genannt. Diese Gene ermöglichen eine leichte Meßbarkeit und Quantifizierbarkeit der Transcriptionsaktivität und damit der Expression der Gene. Damit lassen sich Genomstellen identifizieren, die eine unterschiedliche Produktivität zeigen.

Gemäß einer bevorzugten Ausführungsform umfaßt eine Expressionskassette stromaufwärts, d.h. am 5'-Ende der codierenden Sequenz, einen Promotor und stromabwärts, d.h. am 3'-Ende, ein Polyadenylierungssignal und gegebenenfalls weitere regulatorische Elemente, welche mit der dazwischenliegenden codierenden Sequenz für die Δ6-Acetylenase/Δ6-Desaturase DNA Sequenz operativ verknüpft sind. Unter einer operativen Verknüpfung versteht man die sequenzielle Anordnung von Promotor, codierender Sequenz, Terminator und ggf. weiterer regulativer Elemente derart, daß jedes der regulativen Elemente seine Funktion bei der Expression der codierenden Sequenz bestimmungsgemäß erfüllen kann. Die zur operativen Verknüpfung bevorzugten Sequenzen sind Targeting-Sequenzen zur Gewährleistung der subzellulären Lokalisation in Plastiden. Aber auch Targeting-Sequenzen zur Gewährleistung der subzellulären Lokalisation im Mitochondrium, im Endoplasmatischen Retikulum (= ER), im Zellkern, in Ölkörperchen oder anderen Kompartimenten sind bei Bedarf einsetzbar sowie Translationsverstärker wie die 5'-Führungssequenz aus dem Tabak-Mosaik-Virus (Gallie et al., Nucl. Acids Res. 15 (1987), 8693 -8711).

Eine Expressionskassette kann beispielsweise einen konstitutiven Promotor (bevorzugt den USP- oder Napin-Promotor), das zu exprimierende Gen und das ER-Retentionssignal enthalten. Als ER-Retentionssignal wird bevorzugt die Aminosäuresequenz KDEL (Lysin, Asparaginsäure, Glutaminsäure, Leucin) verwendet.

Die Expressionskassette wird zur Expression in einem prokaryontischen oder eukaryontischen Wirtsorganismus beispielsweise einem Mikroorganismus wie einem Pilz oder einer Pflanze vorteilhafterweise in einen Vektor wie beispielsweise einem Plasmid, einem Phagen oder sonstiger DNA inseriert, der eine optimale Expression der Gene im Wirtsorganismus ermöglicht. Geeignete Plasmide sind beispielsweise in E. coli pLG338, pACYC184, pBR-Serie wie z.B. pBR322, pUC-Serie wie pUC18 oder pUC19, M113mp-Serie, pKC30, pRep4, pHS1, pHS2, pPLc236, pMBL24, pLG200, pUR290, pIN-III¹¹³-B1, λgt11 oder pBdCI, in Streptomyces pIJ101, pIJ364, pIJ702 oder pIJ361, in Bacillus pUB110, pC194 oder pBD214, in Corynebacterium pSA77 oder pAJ667, in Pilzen pALS1, pIL2 oder pBB116, weitere vorteilhafte Pilzvektoren werden von Romanos, M.A. et al., [(1992) "Foreign gene expression in yeast: a review", *Yeast* 8: 423-488] und von van den Hondel, C.A.M.J.J. et al. [(1991) "Heterologous gene expression in filamentous fungi] sowie in More Gene Manipulations in Fungi [J.W. Bennet & L.L. Lasure, eds., p. 396-428: Academic Press: San Diego] und in "Gene transfer systems and vector development for filamentous fungi" [van den Hondel, C.A.M.J.J. & Punt, P.J. (1991) in: Applied Molecular Genetics of Fungi, Peberdy, J.F. et al., eds., p. 1-28, Cambridge University Press: Cambridge] beschrieben. Vorteilhafte Hefepromotoren sind beispielsweise 2∝M, pAG-1, YEp6, YEp13 oder pEMBLYe23. Beispiele für Algen- oder Pflanzenpromotoren sind pLGV23, pGH1ac⁺, pBIN19, pAK2004, pVKH oder pDH51 (siehe Schmidt, R. and Willmitzer, L., 1988). Die oben genannten Vektoren oder Derivate der vorstehend genannten Vektoren stellen eine kleine Auswahl der möglichen Plasmide dar. Weitere Plasmide sind dem Fachmann wohl bekannt und können beispielsweise aus dem Buch Cloning Vectors (Eds. Pouwels P.H. et al. Elsevier, Amsterdam-New York-Oxford, 1985 , ISBN 0 444 904018) entnommen werden. Geeignete pflanzliche Vektoren werden unter anderem in "Methods in Plant Molecular Biology and Biotechnology" (CRC Press), Kap. 6/7, S.71-119 beschrieben. Vorteilhafte Vektoren sind sog. shuttle-Vektoren oder binäre Vektoren, die in E. coli und Agrobacterium replizieren.

Unter Vektoren sind außer Plasmiden auch alle anderen dem Fachmann bekannten Vektoren wie beispielsweise Phagen, Viren wie SV40, CMV, Baculovirus, Adenovirus, Transposons, IS-Elemente, Phasmide, Phagemide, Cosmide, lineare oder zirkuläre DNA zu verstehen. Diese Vektoren können autonom im Wirtsorganismus repliziert oder chromosomal repliziert werden bevorzugt ist eine chromosomale Replikation.

In einer weiteren Ausgestaltungsform des Vektors kann die erfindungsgemäße Expressionskassette auch vorteilhafterweise in Form einer linearen DNA in die Organismen eingeführt werden und über heterologe oder homologe Rekombination in das Genom des Wirtsorganismus integriert werden. Diese lineare DNA kann aus einem linearisierten Plasmid oder nur aus der Expressionskassette als Vektor oder den erfindungsgemäßen Nukleinsäuresequenzen bestehen.

In einer weiteren vorteilhaften Ausführungsform kann die erfindungsgemäße Nukleinsäuresequenz auch alleine in einen Organismus eingebracht werden.

Sollen neben der erfindungsgemäßen Nukleinsäuresequenz weitere Gene in den Organismus eingeführt werden, so können alle zusammen mit einem Reportergen in einem einzigen Vektor oder jedes einzelne Gen mit einem Reportergen in je einem Vektor in den Organismus eingebracht werden, wobei die verschiedenen Vektoren gleichzeitig oder sukzessive eingebracht werden können.

Der Vektor enthält vorteilhaft mindestens eine Kopie der erfindungsgemäßen Nukleinsäuresequenzen und/oder der erfindungsgemäßen Expressionskassette.

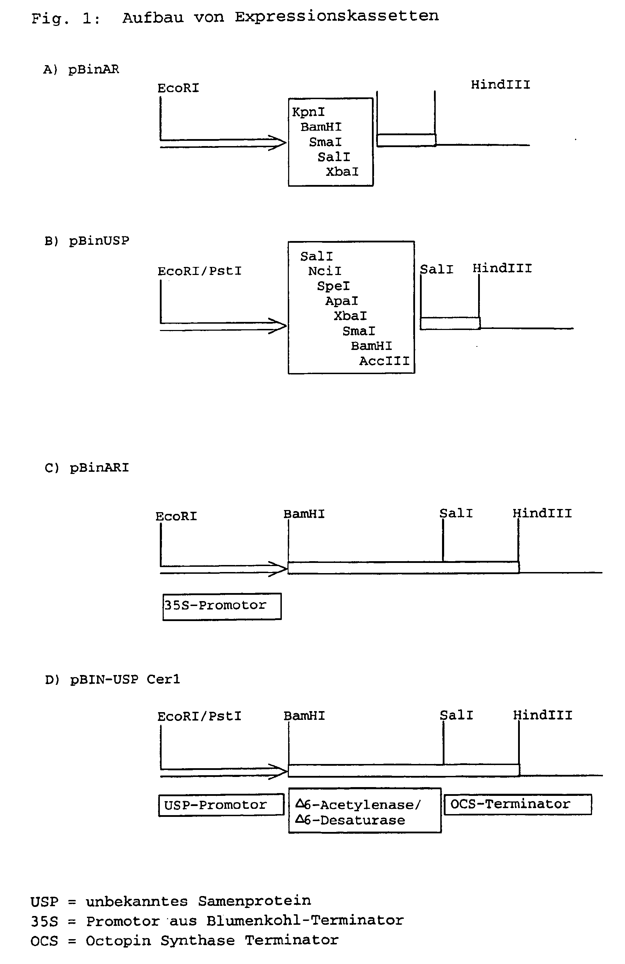

Beispielhaft kann die pflanzliche Expressionskassette in den Tabak-Transformationsvektor pBinAR eingebaut werden. Fig. 1 zeigt die Tabaktransformationsvektoren pBinAR-mit 35S-Promotor (C) bzw. pBin-USP mit dem USP-Promotor (D). Die Ausgangsvektoren sind in Fig. 1 A) und B) dargestellt.

Alternativ kann ein rekombinanter Vektor (= Expressionsvektor) auch in-vitro transkribiert und translatiert werden, z.B. durch Nutzung des T7 Promotors und der T7 RNA Polymerase.

In Prokaryoten verwendete Expressionsvektoren nutzen häufig induzierbare Systeme mit und ohne Fusionsproteinen bzw Fusionsoligopeptiden, wobei diese Fusionen sowohl N-terminal als auch C-terminal oder anderen nutzbaren Domänen eines Proteins erfolgen können. Solche Fusionsvektoren dienen in der Regel dazu: i.) die Expressionsrate der RNA zu erhöhen ii.) die erzielbare Proteinsyntheserate zu erhöhen, iii.) die Löslichkeit des Proteins zu erhöhen, iv.) oder die Reinigung durch einen für die Affinitätschromatographie nutzbare Bindesequenz zu vereinfachen. Häufig werden auch proteolytische Spaltstellen über Fusionsproteine eingeführt, was die Abspaltung eines Teils des Fusionsproteins auch der Reinigung ermöglicht. Solche Erkennungssequenzen für Proteasen erkennen sind z.B. Faktor Xa, Thrombin und Enterokinase.

Typische vorteilhafte Fusions- und Expressionsvektoren sind pGEX [Pharmacia Biotech Inc; Smith, D.B. and Johnson, K.S. (1988) Gene 67: 31-40], pMAL (New England Biolabs, Beverly, MA) and pRIT5 (Pharmacia, Piscataway, NJ) welches Glutathion S-transferase beinhaltet (GST), Maltose Bindeprotein, oder Protein A.

Weitere Beispiele für E. coli Expressionsvektoren sind pTrc [A-mann et al., (1988) *Gene* 69:301-315] und pET Vektoren [Studier et al., Gene Expression Technology: Methods in Enzymology 185, Academic Press, San Diego, California (1990) 60-89; Stratagene, Amsterdam, Niederlande].

Weitere vorteilhafte Vektoren zur Verwendung in Hefe sind pYepSec1 (Baldari, et al., (1987) *Embo J.* 6:229-234), pMFa (Kurjan and Herskowitz, (1982) Cell 30:933-943), pJRY88 (Schultz et al., (1987) Gene 54:113-123), and pYES-Derivate (Invitrogen Corporation, San Diego, CA). Vektoren für die Nutzung in filamentösen Pilzen sind beschrieben in: van den Hondel, C.A.M.J.J. & Punt, P.J. (1991) "Gene transfer systems and vector development for filamentous fungi, in: Applied Molecular Genetics of Fungi, J.F. Peberdy, et al., eds., p. 1-28, Cambridge University Press: Cambridge.

Alternativ können auch vorteilhaft Insektenzellexpressionsvektoren genutzt werden z.B. für die Expression in Sf 9 Zellen. Dies sind z.B. die Vektoren der pAc Serie (Smith et al. (1983) *Mol. Cell Biol.* 3:2156-2165) und der pVL series (Lucklow and Summers (1989) *Virology* 170:31-39).

Des weiteren können zur Genexpression vorteilhaft Pflanzenzellen oder Algenzellen genutzt werden. Beispiele für Pflanzenexpressionsvektoren finden sich in Becker, D., et al. (1992) "New plant binary vectors with selectable markers located proximal to the left border", *Plant Mol. Biol.* 20: 1195-1197 oder in Bevan, M.W. (1984) "Binary *Agrobacterium* vectors for plant transformation", *Nucl. Acid. Res.* 12: 8711-8721.

Weiterhin können die erfindungsgemäßen Nukleinsäuresequenzen in Säugerzellen exprimiert werden. Beispiel für entsprechende Expressionsvektoren sind pCDM8 und pMT2PC genannt in: Seed, B. (1987) *Nature* 329:840 oder Kaufman et al. (1987) *EMBO J.* 6: 187-195). Dabei sind vorzugsweise zu nutzende Promotoren viralen Ursprungs wie z.B. Promotoren des Polyoma, Adenovirus 2, Cytomegalovirus oder Simian Virus 40. Weitere prokaryotische und eukaryotische Expressionssysteme sind genannt in Kapitel 16 und 17 in Sambrook et al., *Molecular Cloning: A Laboratory Manual. 2nd, ed., Cold Spring Harbor Laboratory,* Cold Spring Harbor Laboratory Press, Cold Spring Harbor, NY, 1989.

Das Einbringen der erfindungsgemäßen Nukleinsäuren, der Expressionskassette oder des Vektors in Organismen beispielsweise in Pflanzen kann prinzipiell nach allen dem Fachmann bekannten Methoden erfolgen.

Für Mikroorganismen kann der Fachmann entsprechende Methoden den Lehrbüchern von Sambrook, J. et al. (1989) Molecular cloning: A laboratory manual, Cold Spring Harbor Laboratory Press, von F.M. Ausubel et al. (1994) Current protocols in molecular biology, John Wiley and Sons, von D.M. Glover et al., DNA Cloning Vol.1, (1995), IRL Press (ISBN 019-963476-9), von Kaiser et al. (1994) Methods in Yeast Genetics, Cold Spring Habor Laboratory Press oder Guthrie et al. Guide to Yeast Genetics and Molecular Biology, Methods in Enzymology, 1994, Academic Press entnehmen.

Die Übertragung von Fremdgenen in das Genom einer Pflanze wird als Transformation bezeichnet. Es werden dabei die beschriebenen Methoden zur Transformation und Regeneration von Pflanzen aus Pflanzengeweben oder Pflanzenzellen zur transienten oder stabilen Transformation genutzt. Geeignete Methoden sind die Protoplastentransformation durch Polyethylenglykol-induzierte DNA-Aufnahme, das biolistische Verfahren mit der Genkanone - die sogenannte particle bombardment Methode, die Elektroporation, die Inkubation trockener Embryonen in DNA-haltiger Lösung, die Mikroinjektion und der durch Agrobacterium vermittelte Gentransfer. Die genannten Verfahren sind beispielsweise in B. Jenes et al., Techniques for Gene Transfer, in: Transgenic Plants, Vol. 1, Engineering and Utilization, herausgegeben von S.D. Kung und R. Wu, Academic Press (1993) 128-143 sowie in Potrykus Annu. Rev. Plant Physiol. Plant Molec. Biol. 42 (1991) 205-225) beschrieben. Vorzugsweise wird das zu exprimierende Konstrukt in einen Vektor kloniert, der geeignet ist, Agrobacterium tumefaciens zu transformieren, beispielsweise pBin19 (Bevan et al., Nucl. Acids Res. 12 (1984) 8711). Mit einem solchen Vektor transformierte Agrobakterien können dann in bekannter Weise zur Transformation von Pflanzen, insbesondere von Kulturpflanzen, wie z.B. von Tabakpflanzen, verwendet werden, indem beispielsweise verwundete Blätter oder Blattstücke in einer Agrobakterienlösung gebadet und anschließend in geeigneten Medien kultiviert werden. Die Transformation von Pflanzen mit Agrobacterium tumefaciens wird beispielsweise von Höfgen und Willmitzer in Nucl. Acid Res. (1988) 16, 9877 beschrieben oder ist unter anderem bekannt aus F.F. White, Vectors for Gene Transfer in Higher Plants; in Transgenic Plants, Vol. 1, Engineering and Utilization, herausgegeben von S.D. Kung und R. Wu, Academic Press, 1993, S. 15-38.

Mit einem erfindungsgemäßen Expressionsvektor transformierte Agrobakterien können ebenfalls in bekannter Weise zur Transformation von Pflanzen wie Testpflanzen wie Arabidopsis oder Kulturpflanzen wie Getreide, Mais, Hafer, Roggen, Gerste, Weizen, Soja, Reis, Baumwolle, Zuckerrübe, Canola, Sonnenblume, Flachs, Hanf, Kartoffel, Tabak, Tomate, Karotte, Paprika, Raps, Tapioka, Maniok, Pfeilwurz, Tagetes, Alfalfa, Salat und den verschiedenen Baum-, Nuß- und Weinspezies, insbesondere von Öl-haltigen Kulturpflanzen, wie Soja, Erdnuß, Rizinus, Sonnenblume, Mais, Baumwolle, Flachs, Raps, Kokosnuß, Ölpalme, Färbersaflor (Carthamus tinctorius) oder Kakaobohne verwendet werden, z.B. indem verwundete Blätter oder Blattstücke in einer Agrobakterienlösung gebadet und anschließend in geeigneten Medien kultiviert werden.

Die genetisch veränderten Pflanzenzellen können über alle dem Fachmann bekannten Methoden regeneriert werden. Entsprechende Methoden können den oben genannten Schriften von S.D. Kung und R. Wu, Potrykus oder Höfgen und Willmitzer entnommen werden.

Als Organismen bzw. Wirtsorganismen für die erfindungsgemäße Nukleinsäure, die Expressionskassette oder den Vektor eignen sich prinzipiell vorteilhaft alle Organismen, die in der Lage sind Fettsäuren speziell ungesättigte Fettsäuren zu synthetisieren bzw. für die Expression rekombinanter Gene geeignet sind. Beispielhaft seien Pflanzen wie Arabidopsis, Asteraceae wie Calendula oder Kulturpflanzen wie Soja, Erdnuß, Rizinus, Sonnenblume, Mais, Baumwolle, Flachs, Raps, Kokosnuß, Ölpalme, Färbersaflor (Carthamus tinctorius) oder Kakaobohne, Mikroorganismen wie Pilze beispielsweise die Gattung Mortierella, Saprolegnia oder Pythium, Bakterien wie die Gattung Escherichia, Hefen wie die Gattung Saccharomyces, Cyanobakterien, Ciliaten, Algen oder Protozoen wie Dinoflagellaten wie Crypthecodinium genannt. Bevorzugt werden Organismen, die natürlicherweise Öle in größeren Mengen synthetisieren können wie Pilze wie Mortierella alpina, Pythium insidiosum oder Pflanzen wie Soja, Raps, Kokosnuß, Ölpalme, Färbersaflor, Rizinus, Calendula, Erdnuß, Kakaobohne oder Sonnenblume oder Hefen wie Saccharomyces cerevisiae, besonders bevorzugt werden Soja, Raps, Sonnenblume, Calendula oder Saccharomyces cerevisiae. Prinzipiell sind als Wirtsorganismen auch transgene Tiere geeignet beispielsweise C. elegans.

Nutzbare Wirtszellen sind weiterhin genannt in: Goeddel, *Gene Expression Technology: Methods in Enzymology* 185, Academic Press, San Diego, CA (1990).

verwendbare Expressionsstämme z.B. solche, die eine geringere Proteaseaktivität aufweisen sind beschrieben in: Gottesman, S., *Gene Expression Technology: Methods in Enzymology* 185, Academic Press, San Diego, California (1990) 119-128.

Ein weiterer Gegenstand der Erfindung betrifft die Verwendung einer Expressionskassette enthaltend DNA-Sequenzen codierend für ein Δ6-Acetylenase/Δ6-DesaturaseGen oder mit diesen hybridisierende DNA-Sequenzen zur Transformation von Pflanzenzellen, -geweben oder Pflanzenteilen. Ziel der Verwendung ist die Erhöhung des Gehaltes an Fettsäuren, Ölen oder Lipiden mit erhöhtem Gehalt an Dreifachbindungen und Doppelbindung in Δ6-Position.

Dabei kann je nach Wahl des Promotors die Expression des Δ6-Acetylenase/Δ6-Desaturase-Gens spezifisch in den Blättern, in den Samen, den Knollen oder anderen Teilen der Pflanze erfolgen. Solche Fettsäuren, Öle oder Lipide mit Δ6-Dreifachbindungen oder Δ6-Doppelbindungen überproduzierenden transgenen Pflanzen, deren Vermehrungsgut, sowie deren Pflanzenzellen, -gewebe oder -teile, sind ein weiterer Gegenstand der vorliegenden Erfindung. Ein bevorzugter erfindungsgemäßer Gegenstand sind transgene Pflanze enthaltend eine erfindungsgemäße funktionelle oder nicht funktionelle (= Antisense-DNA oder enzymatische inaktives Enzym) Nukleinsäuresequenz oder eine funktionelle oder nicht funktionelle Expressionskassette.

Die Expressionskassette oder die erfindungsgemäßen Nukleinsäuresequenzen enthaltend eine Δ6-Acetylenase/Δ6-Desaturasegensequenz kann darüber hinaus auch zur Transformation der oben beispielhaft genannten Organismen wie Bakterien, Cyanobakterien, Hefen, filamentösen Pilzen, Ciliaten und Algen mit dem Ziel einer Erhöhung des Gehaltes an Fettsäuren, Ölen oder Lipiden mit Δ6-Dreifachbindungen oder Δ6-Doppelbindungen eingesetzt werden.

Erhöhung des Gehaltes von Fettsäuren, Ölen oder Lipiden mit Δ6-Dreifachbindungen oder Δ6-Doppelbindungen bedeutet im Rahmen der vorliegenden Erfindung beispielsweise die künstlich erworbene Fähigkeit einer erhöhten Biosyntheseleistung durch funktionelle Überexpression des Δ6-Acetylenase/Δ6-Desaturase-Gens in den erfindungsgemäßen nicht-humanen Organismen vorteilhaft in den erfindungsgemäßen transgenen Pflanzen gegenüber den nicht gentechnisch modifizierten Ausgangspflanzen zumindest für die Dauer mindestens einer Pflanzengeneration.

Der Biosyntheseort von Fettsäuren, Ölen oder Lipiden beispielsweise ist im allgemeinen der Samen oder Zellschichten des Samens, so daß eine samenspezifische Expression des Δ6-Acetylenase/Δ6-Desaturase-Gens sinnvoll ist. Es ist jedoch naheliegend, daß die Biosynthese von Fettsäuren, Ölen oder Lipiden nicht auf das Samengewebe beschränkt sein muß, sondern auch in allen übrigen Teilen der Pflanze - beispielsweise in Epidermiszellen oder in den Knollen - gewebespezifisch erfolgen kann.

Darüberhinaus ist eine konstitutive Expression des exogenen Δ6-Acetylenase/Δ6-Desaturase-Gens von Vorteil. Andererseits kann aber auch eine induzierbare Expression wünschenswert erscheinen.

Die Wirksamkeit der Expression des Transgens Δ6-Acetylenase/Δ6-Desaturase-Gens kann beispielsweise *in vitro* durch Sproßmeristemvermehrung ermittelt werden. Zudem kann eine in Art und Höhe veränderte Expression des Δ6-Acetylenase/Δ6-Desaturase-Gens und deren Auswirkung auf die Fettsäure-, Öl- oder Lipidbiosyntheseleistung an Testpflanzen in Gewächshausversuchen getestet werden.

Gegenstand der Erfindung sind außerdem transgene Pflanzen, transformiert mit einer Expressionskassette enthaltend eine Δ6-Acetylenase/Δ6-Desaturase-Gensequenz
oder mit dieser hybridisierende DNA-Sequenzen, sowie transgene Zellen, Gewebe, Teile und Vermehrungsgut solcher Pflanzen. Besonders bevorzugt sind dabei transgene Kulturpflanzen, wie z.B. Gerste, Weizen, Roggen, Hafer, Mais, Soja, Reis, Baumwolle, Zuckerrübe, Raps und Canola, Sonnenblume, Flachs, Hanf, Kartoffel, Tabak, Tomate, Raps, Tapioka, Maniok, Pfeilwurz, Alfalfa, Salat und die verschiedenen Baum-, Nuß- und Weinspezies.

Pflanzen im Sinne der Erfindung sind mono- und dikotyle Pflanzen oder Algen.

Eine weitere erfindungsgemäße Ausgestaltung sind wie oben beschriebenen transgene Pflanzen, die eine funktionelle oder nicht funktionelle erfindungsgemäße Nukleinsäuresequenz oder eine funktionelle oder nicht funktionelle erfindungsgemäße Expressionskassette enthalten. Unter nicht funktionell ist
zu verstehen, daß kein enzymatisch aktives Protein mehr synthetisiert wird. Außerdem ist unter nicht funktionellen Nukleinsäuren oder Nukleinsäurekonstrukten auch eine sogenannte Antisense-DNA zu verstehen, die zu transgenen Pflanzen führt, die eine Reduktion der enzymatischen Aktivität oder keine enzymatischen Aktivität aufweisen. Mit Hilfe der Antisense-Technik, speziell wenn die erfindunsgemäße Nukleinsäuresequenz mit anderen Fettsäuresynthesegene in der Antisense-DNA kombiniert wird, ist es möglich Triglyceride mit einem erhöhten Gehalt an gesättigten Fettsäuren bzw. gesättigte Fettsäuren zu synthetisieren. Unter transgenen Pflanzen sind einzelne Pflanzenzellen und deren Kulturen auf Festmedien oder in Flüssigkultur, Pflanzenteile und ganze Pflanzen zu verstehen.

Weitere Gegenstände der Erfindung sind:
- Verfahren zur Transformation einer Pflanze dadurch gekennzeichnet, daß man Expressionskassetten enthaltend eine Δ6-Acetylenase/Δ6-Desaturase-Gensequenz oder mit dieser hybridisierende DNA'-Sequenzen in eine Pflanzenzelle, in Kallusgewebe, eine ganze Pflanze oder Protoplasten von Pflanzen einbringt.
- Verwendung einer Δ6-Acetylenase/Δ6-Desaturase-DNA-Gensequenz oder mit dieser hybridisierende DNA-Sequenzen zur Herstellung von Pflanzen mit erhöhtem Gehalt an Fettsäuren, Ölen oder Lipiden mit Dreifachbindungen oder delta-6-Doppelbindungen durch Expression dieser D6-Acetylenase/Desaturase DNA-Sequenz in Pflanzen.

Ein weiterer erfindungsgemäßer Gegenstand ist ein Verfahren zur Herstellung von ungesättigten Fettsäuren, dadurch gekennzeichnet, daß man mindestens eine oben beschriebene erfindungsgemäße Nukleinsäuresequenz oder mindestens ein erfindungsgemäßes Nukleinsäurekonstrukt in einen bevorzugt Öl produzierenden nicht-humanen Organismus bringt, diesen Organismus anzieht und daß in dem Organismus enthaltene Öl isoliert und die im Öl enthaltenden Fettsäuren freisetzt. Diese ungesättigten Fettsäuren enthalten vorteilhaft Δ6-Dreifach- und/oder Δ6-Doppelbindungen. Die Fettsäuren können aus den Ölen bzw. Lipiden beispielsweise über eine basische Hydrolyse z.B. mit NaOH oder KOH freigesetzt werden.

Auch ein Verfahren zur Herstellung von Triglyceriden mit einem erhöhten Gehalt an ungesättigten Fettsäuren, dadurch gekennzeichnet, daß man mindestens eine oben beschriebene erfindungsgemäße Nukleinsäuresequenz oder mindestens eine erfindungsgemäße Expressionskassette in einen Öl produzierenden nicht-humanen Organismus bringt, diesen Organismus anzieht und daß in dem Organismus enthaltene Öl isoliert, gehört zu den Erfindungsgegenständen.

Ein weiterer erfindungsgemäßer Gegenstand ist ein Verfahren zur Herstellung von Triglyceriden mit einem erhöhten Gehalt an ungesättigten Fettsäuren, indem man Triglyceride mit gesättigten oder ungesättigten oder gesättigten und ungesättigten Fettsäuren mit mindestens einem der Protein, das durch eine der Sequenzen SEQ ID NO: 1 oder SEQ ID NO: 3 kodiert wird, inkubiert.. Vorteilhaft wird das Verfahren in Gegenwart von Verbindungen durchgeführt, die Reduktionsäquivalente aufnehmen oder abgeben können. Anschließend können die Fettsäuren aus den Triglyceriden freigesetzt werden.

Die oben genannten Verfahren ermöglichen vorteilhaft die Synthese von Fettsäuren oder Triglyceriden mit einem erhöhten Gehalt an Fettsäuren mit Δ6-Dreifach- und/oder Δ6-Doppelbindungen.

Mit Hilfe der sogenannten Antisense-Technologie können in einem Verfahren auch Fettsäuren oder Triglyceride mit einem erhöhten Gehalt an gesättigten Fettsäuren hergestellt werden.

Als Organismen für die genannten Verfahren seien beispielhaft Pflanzen wie Arabidopsis, Gerste, Weizen, Roggen, Hafer, Mais, Soja, Reis, Baumwolle, Zuckerrübe, Raps und Canola, Sonnenblume, Flachs, Hanf, Kartoffel, Tabak, Tomate, Raps, Tapioka, Maniok, Pfeilwurz, Alfalfa, Erdnuß, Rizinus, Kokosnuß, Ölpalme, Färbersaflor (Carthamus tinctorius) oder Kakaobohne, Mikroorganismen wie Pilze Mortierella, Saprolegnia oder Pythium, Bakterien wie die Gattung Escherichia, Cyanobakterien, Hefen wie die Gattung Saccharomyces, Algen oder Protozoen wie Dinoflagellaten wie Crypthecodinium genannt. Bevorzugt werden Organismen, die natürlicherweise Öle in größeren Mengen synthetisieren können wie Mikroorganismen wie Pilze wie Mortierella alpina, Pythium insidiosum oder Pflanzen wie Soja, Raps, Kokosnuß, Ölpalme, Färbersaflor, Rizinus, Calendula, Erdnuß, Kakaobohne oder Sonnenblume oder Hefen wie Saccharomyces cerevisiae, besonders bevorzugt werden Soja, Raps, Sonnenblume, Carthamus oder Saccharomyces cerevisiae.

Die in den Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0°C und 100°C, bevorzugt zwischen 10°C bis 60°C unter Sauerstoffbegasung angezogen. Dabei kann der pH der Nährflüssigkeit auf einen festen Wert gehalten werden, das heißt während der Anzucht reguliert werden oder nicht. Die Anzucht kann batch weise, semi batch weise oder kontinuierlich erfolgen. Nährstoffe können zu beginn der Fermentation vorgelegt oder semikontinuierlich oder kontinuierlich nach gefüttert werden.

Pflanzen werden nach Transformation zunächst wie oben beschrieben regeneriert und anschließend wie üblich angezüchtet bzw. angebaut.

Aus den Organismen werden nach Anzucht die Lipide in üblicher Weise gewonnen. Hierzu können die Organismen nach Ernte zunächst aufgeschlossen werden oder direkt verwendet werden. Die Lipide werden vorteilhaft mit geeigneten Lösungsmitteln wie apolare Lösungsmittel wie Hexan oder Ethanol, Isopropanol oder Gemischen wie Hexan/Isopropanol, Phenol/Chloroform/Isoamylalkohol bei Temperaturen zwischen 0°C bis 80°C, bevorzugt zwischen 20°C bis 50°C extrahiert. Die Biomasse wird in der Regel mit einem Überschuß an Lösungsmittel extrahiert beispielsweise einem Überschuß von Lösungmittel zu Biomasse von 1:4. Das Lösungsmittel wird anschließend beispielsweise über eine Destillation entfernt.
Die Extraktion kann auch mit superkritischem CO₂ erfolgen. Nach Extraktion kann die restliche Biomasse beispielsweise über Filtration entfernt werden.

Das so gewonnene Rohöl kann anschließend weiter aufgereinigt werden, beispielsweise in dem Trübungen über das Versetzen mit polaren Lösungsmittel wie Aceton oder Chloroform und anschließender Filtration oder Zentrifugation entfernt werden. Auch eine weitere Reinigung über Säulen ist möglich.

Zur Gewinnung der freien Fettsäuren aus den Triglyceriden werden diese in üblicherweise verseift.

Weiter hin werden ungesättigte Fettsäuren sowie Trigylceride mit einem erhöhten Gehalt an ungesättigten Fettsäuren, die nach den oben genannten Verfahren hergestellt wurden, sowie deren Verwendung zur Herstellung von Nahrungsmitteln, Tierfutter, Kosmetika oder Pharmazeutika, beschrieben. Hierzu werden diese den Nahrungsmitteln, dem Tierfutter, den Kosmetika oder Pharmazeutika in üblichen Mengen zugesetzt.

Die Erfindung wird durch die folgenden Beispiele näher erläutert:

### Beispiele

### Beispiel 1:

### Allgemeine Klonierungsverfahren:

Die Klonierungsverfahren wie z.B. Restriktionsspaltungen, Agarose-Gelelektrophorese, Reinigung von DNA-Fragmenten, Transfer von Nukleinsäuren auf Nitrozellulose und Nylon Membranen, Verknüpfen von DNA-Fragmenten, Transformation von Escherichia coli Zellen, Anzucht von Bakterien und die Sequenzanalyse rekombinanter DNA wurden wie bei Sambrook et al. (1989) (Cold Spring Harbor Laboratory Press: ISBN 0-87969-309-6) beschrieben durchgeführt.

### Beispiel 2:

### Sequenzanalyse rekombinanter DNA:

Die Sequenzierung rekombinanter DNA-Moleküle erfolgte mit einem Laserfluoreszenz-DNA-Sequenzierer der Firma ABI nach der Methode von Sanger (Sanger et al. (1977) Proc. Natl. Acad. Sci. USA74, 5463-5467). Fragmente resultierend aus einer Polymerase Kettenreaktion wurden zur Vermeidung von Polymerasefehlern in zu exprimierenden Konstrukten sequenziert und überprüft.

### Beispiel 3:

### Erzeugung transgener Rapspflanzen (verändert nach Moloney et al., 1992, Plant Cell Reports, 8:238-242)

Zur Erzeugung transgener Rapspflanzen wurden binäre Vektoren in Agrobacterium tumefaciens C58C1:pGV2260 oder Escherichia coli genutzt (Deblaere et al, 1984, Nucl. Acids. Res. 13, 4777-4788). Zur Transformation von Rapspflanzen (Var. Drakkar, NPZ Nordeutsche Pflanzenzucht, Hohenlieth, Deutschland), wurde eine 1:50 Verdünnung einer Übernachtkultur einer positiv transformierten Agrobakterienkolonie in Murashige-Skoog Medium (Murashige und Skoog 1962 Physiol. Plant. 15, 473) mit 3 % Saccharose (3MS-Medium) benutzt. Petiolen oder Hypokotyledonen frisch gekeimter steriler Rapspflanzen (zu je ca. 1 cm²) wurden in einer Petrischale mit einer 1:50 Agrobakterienverdünnung für 5-10 Minuten inkubiert. Es folgt eine 3-tägige Conkubation in Dunkelheit bei 25_C auf 3MS-Medium mit 0,8 % Bacto-Agar. Die Kultivierung wurde nach 3 Tagen mit 16 Stunden Licht / 8 Stunden Dunkelheit weitergeführt und in wöchentlichem Rhythmus auf MS-Medium mit 500 mg/l Claforan (Cefotaxime-Natrium), 50 mg/l Kanamycin, 20 mikroM Benzylaminopurin (BAP) und 1,6 g/l Glukose weitergeführt. Wachsende Sprosse wurden auf MS-Medium mit 2 % Saccharose, 250 mg/l Claforan und 0,8 % Bacto-Agar überführt. Bildeten sich nach drei Wochen keine Wurzeln, so wurde als Wachstumshormon 2-Indolbuttersäure zum Bewurzeln zum Medium gegeben.

### Beispiel 4:

### Erzeugung transgener Arabidopsis thaliana Pflanzen

Die Transformation von Arabidopsis thaliana Var. Columbia Col 0 (Lehle Seeds, Round Rock, Texas, USA) erfolgte mittels Blüteninfiltrationsmethode wie beschrieben bei: Bechtold, N., Ellis, J. and Pelletier, G. in Planta, Agrobacterium mediated gene transfer by infiltration of adult Arabidopsis thaliana plants, C.R. Acad. Sci. Paris, Life Sciences 316 (1993), 1194-119 oder mittels Wurzeltransformationsmethode.

### Beispiel 5:

Die Transformation von Maispflanzen erfolgte wie bei Pareddy, D., Petolino, J., Skokut, T., Hopkins, N., Miller, M., Welter, M., Smith, K., Clayton, D., Pescitelli, S., Gould, A., Maize Transformation via Helium Blasting. Maydica. 42(2): 143-154, 1997, beschrieben.

### Beispiel 6:

### Isolierung und Klonierung der Δ6-Acetylenase/Δ6-Desaturase und Δ6-Desaturase aus Ceratodon purpureus

Um DNA-Sequenzen aus Ceratodon purpureus zu isolieren, die für eine Δ6-Acetylenase/Δ6-Desaturase und eine Δ6-Desaturase kodieren, wurden verschiedene degenerierte Oligonukleotidprimer von DNA-Sequenzen abgeleitet, die für Δ5- (EMBL Accession-Nr. Z81122) und Δ6-Fettsäure-Desaturasen (U79010, AJ222980, AF031477 kodieren:

| | |
|---|---|
| Primer A: | 5'-TGG TGG AA(A/G) TGG A(A/C)I CA(C/T) AA-3' |
| | forward Primer, abgeleitet von der Aminosäuresequenz WWKW(N/T/K)H(N/K) |
| Primer B: | 5'-(T/G)GI TGG AA(A/G) (T/G)(G/A)I (A/C)AI CA(C/T) AA-3' |
| | forward Primer, abgeleitet von der Aminosäuresequenz (G/W)WK(E/D/W) (N/Q/K)H(N/K) |
| Primer C: | 5'-AT (A/T/G/C)T(T/G) (A/T/G/C)GG (A/G)AA (A/T/G/C)A(A/G) (A/G)TG (A/G)TG -3', reverse primer, abgeleitet von der Aminosäuresequenz (I/M)(H/Q/N)PF(L/F)HH |

Mittels Polymerasekettenreaction (PCR) mit Einzelstrang-cDNA aus C. purpureus wurden mit Primer A und Primer C zwei DNA-Fragmente von 557 bp (Cer3) und 575 bp (Cer16) Länge und mit Primer B und Primer C ein DNA-Fragment von 560 bp (Cer1) Länge amplifiziert. Es wurde folgendes Programm für die Amplifizierung benutzt: 10 min bei 94°C, Pause für 'hot start' bei 72°C, gefolgt von 32 Zyklen von 20 s bei 94°C, 1 min bei 45°C (Bindungstemperatur, Tₘ) und 1 min bei 72°C, 1 Zyklus von 10 min bei 72°C und Stop bei 4°C. Für die Amplifikation wurde die *Taq*-DNA-Polymerase (Gibco BRL) verwendet.

Die oben genannten doppelsträngigen DNA-Fragmente aus den zwei PCR-Amplifikationen wurden in den pGEM-T Vektor (Promega) legiert, in *E. coli* XL1blue MRF' Kan (Stratagene) transformiert und mit dem ABI PRISM Big Dye Terminator Cycle Sequencing Ready Reaction Kit (Perkin-Elmer, Weiterstadt) sequenziert. Die DNA-Teilsequenzen von Cer1 und Cer3 zeigten 70 % Identität. Die oben genannten DNA-Teilsequenzen kodierten ohne Primer für offene Leserahmen bei Cer1 von 173 Aminosäuren (SEQ ID NO: 5 = Partielle Nukleotidsequenz ohne Primer von Cer1 und SEQ ID NO: 6 = Partielle deduzierte Aminosäuresequenz von Cer1), bei Cer 3 von 172 Aminosäuren (SEQ ID NO: 7 = Partielle Nukleotidsequenz ohne Primer von Cer3 und SEQ ID NO: 8 = Partielle deduzierte Aminosäuresequenz von Cer3) und bei Cer16 von 178 Aminosäuren (SEQ ID NO: 9 = Partielle Nukleotidsequenz ohne Primer von Cer16 und SEQ ID NO: 10 = Partielle deduzierte Aminosäuresequenz von Cer16). Die abgeleitete Proteinsequenz von Cer1 wies 64 % zu Cer3 und 28 % identische Aminosäuren zu Cer16 auf; Cer 3 und Cer16 wiesen wiederum 27 % identische Aminosäuren auf.

Die höchste Ähnlichkeit der Cer1- und Cer3-Proteine besteht zu der Δ6-Acyllipid-Desaturase aus Physcomitrella patens (Girke et al., Plant J., 15, 1998: 39-48), während Cer16 die höchste Ähnlichkeit zu der Δ6-Acyllipid-Desaturase und der Δ8-Sphingolipid-Desaturase aus höheren Pflanzen aufweist.

Eine gerichtete λZAP-cDNA-Bank von Ceratodon purpureus wurde von Fritz Thummler, Botanisches Institut der Universität München, zur Verfügung gestellt (Pasentsis et al., Plant J., 13, 1, 1998: 51-61). Es wurde ein PCR-Test dieser Ceratodon-Bank durchgeführt, bei dem spezifische Primer von den oben genannten DNA-Teilsequenzen Cer1, Cer3 und Cer16 abgeleitet wurden:

Spezifische forward und reverse Primer:

| | |
|---|---|
| Cer1: | 5'-CGAATGAGTGCGACGAAC -3' + 5'-AATAACCTGGGCTCTCAC-3' |
| Cer3: | 5'-ATGAGGATATTGATACTCTC-3' + 5'-GCAATCTGGGCATTCACG-3' |
| Cer16: | 5'-GACATCAAAGCTCTTCTC-3' + 5'-GGCGATGAGAAGTGGTTC-3' |

Eine Restriktionsanalyse (Hind III bzw. EcoR V) der aus der cDNA-Bank mittels PCR amplifizierten Produkte zeigte in allen drei Fällen das gleiche Restriktionsmuster wie das der PCR-Amplifikate aus der ss-cDNA, d.h. die Ceratodon-cDNA-Bank enthält die drei Klone Cer1, Cer3 und Cer16.

### Beispiel 7:

### cDNA-Bank Screening und Sequenzierung der "full length" Klone

DNA-Minipräparationen, der drei aus ss-cDNA amplifizierten PCR-Fragmente Cer1, Cer3, Cer16 von -570 bp Länge in pGEM-T (siehe Beispiel 6) wurden für das weitere Screening der vollständigen Klone aus einer λ ZAP-cDNA-Bank von Ceratodon purpureus an M. Lee und S. Stymne abgegeben. Dieses cDNA-Bank-Screening führte bisher zu zwei vollständigen Klonen von Cer1 und Cer3 mit Inserts von ca. 2,2 kb, die als EcoR I / Kpn I-Fragmente aus dem λ ZAP-Vektor in die EcoR I / Kpn I-Schnittstellen des puc19-Vektors (New England Biolabs) subkloniert und in *E*. *coli* JM105 transformiert wurden.

Ein weiteres Screening der cDNA-Bank mit Cer1 und Cer3 als Hybridisierungsproben unter niedriger Stringenz zeigte, daß mindestens ein weiterer Cer1-homologer Klon existiert, der evtl. für die Δ5-Desaturase kodieren könnte.

Zwei E. coli-Klone, Cer1-50 und Cer3-50, wurden vollständig sequenziert. Cer1-50 hat eine Länge von 2003 bp (SEQ ID NO: 1 = Nukleotidsequenz der Δ6-Acetylenase/Δ6-Desaturase aus Ceratodon purpureus mit 5'- und 3'-untranslatierten Regionen und polyA) und kodiert für ein offenes Leseraster von 483 Aminosäuren (SEQ ID NO: 2 = deduzierte Aminosäuresequenz der Δ6-Acetylenase/Δ6-Desaturase aus Ceratodon purpureus). Cer3-50 besitzt eine Länge von 2142 bp (SEQ ID NO: 11 = Nukleotidsequenz [2142 bp] der Δ6-Desaturase aus Ceratodon purpureus mit 5'- und 3'-untranslatierten Regionen) mit einen offenen Leserahmen von 520 Aminosäuren (SEQ ID NO: 12 = deduzierte Aminosäuresequenz der Δ6-Desaturase aus Ceratodon purpureus). Beide Proteinsequenzen weisen N-terminal das hochkonservierte HPGG-Motiv des Cytochrom b₅ auf (Lederer F., Biochimie 76, 1994: 674-692) und C-terminal die für Desaturasen charakteristischen drei Histidin-Boxen auf (Shanklin et al., Biochemistry, 33, 1994: 12787-12794). Sie stellen somit weitere Mitglieder der wachsenden Familie der Cytochrom b₅-Fusionsproteine dar (Napier et al., Trends in PLant Science, 4, 1, 1999: 2-4). Das erste Histidin der dritten Box ist gegen Glutamin ausgetauscht, einem weiterem Charakteristikum von Δ5- und Δ6-Acyllipid-Desaturasen sowie Δ8-Sphingolipid-Desaturasen.

### Beispiel 8:

Klonierung der gesamten funktionellen aktiven Δ6-Acetylenase/Δ6-Desaturase- und Δ6-Desaturase-Sequenz mittels PCR und die Bereitstellung dieser Sequenz für die Klonierung in Vektoren sowie funktionelle Expression in Hefe

Es wurde eine cDNA hergestellt, die für Enzyme mit Δ6-Acetylenase/Δ6-Desaturase-Aktivität aus Ceratodon purpureus codiert. Analog zu dem hier beschriebenen Beispiel wurde die Δ6-Desaturase kloniert (siehe SEQ ID NO: 2, SEQ ID NO: 4 bzw. SEQ ID NO: 12).

Hierzu wird zunächst anhand der Cer1-cDNA für die D6-Acetylenase/Desaturase aus Ceratodon purpureus die Oligonukleotide für eine Polymerase Kettenreakion (PCR) abgeleitet.

| | |
|---|---|
| Cer1: | 5'- CC GGTACC **ATG** GCC CTC GTT ACC GAC-3' + |
| | 5'- CC GAATTC **TTA** GTG AGC GTG AAG CCG-3' |
| Cer3: | 5'- CC GGTACC **ATG** GTG TCC CAG GGC GGC-3' + |
| | 5'- CC GAATTC **TCA** ACT CGC AGC AAG CTG-3' |

Die folgenden Primer wurden abgeleitet von Cer1 für die Expression in Hefe angepaßt:

| | |
|---|---|
| 5'-Primer: | 5'-AAAAGGATCCAAAATGGCCCTCGTTACCGAC-3' |
| 3'-Primer: | 5'-AAAAGTCGACTTAGTGAGCGTGAAGCC-3' |

In einer PCR Reaktion wird eine D6-Acetylenase/Desaturase cDNA aus Ceratodon purpureus als Matrize verwendet. Mithilfe der Primer wird eine BamHI-Restriktionsschnittstelle vor dem Startcodon der D6-Acetylenase/Desaturase cDNA eingeführt. Für eine gerichtete Klonierung wird hinter das Stopcodon eine SalI-Restriktionsschnittstelle eingeführt. Die Reaktionsgemische enthielten ca. 1 ng/micro 1 Matrizen DNA, 0,5 micro M der Oligonukleotide und, 200 microM Desoxy-Nukleotide (Pharmacia), 50 mM KCl, 10 mM Tris-HCl (pH 8,3 bei 25_C, 1,5 mM MgCl₂) und 0,02 U/ micro 1 Pwo Polymerase (Boehringer Mannheim) und werden in einer PCR-Maschine der Firma Perkin Elmer mit folgendem Temperaturprogramm inkubiert:

| | |
|---|---|
| Anlagerungstemperatur: | 50_C, 52 sec |
| Denaturierungstemperatur: | 95_C, 52 sec |
| Elongationstemperatur: | 72_C, 90 sec |
| Anzahl der Zyklen: | 30 |

Das erhaltene Fragment von 1467 Basenpaaren wird in den mit E-coRV gespaltenen Vektor pBluescript SK- (Stratagene) ligiert. Durch Kontrollspaltung wird ein Klon identifiziert pBS-Cer1, dessen Insert durch BamHI/SalI in voller Länge exzisierbar ist (1452 Basenpaare plus 15 Nukleotide Restriktionsschnittstellen) und folgende Sequenz aufweist (das Start- und Stopcodon ist unterstrichen, die Schnittstellen sind kursiv dargestellt). Analog kann auch eine cDNA Sequenz des Klones Cer50 genutzt werden. Dabei handelt es sich um eine monofunktionelle delta-6-Desaturase (siehe SEQ ID NO: 3). Die abgeleitete Aminosäuresequenz ist SEQ ID NO: 4 zu entnehmen.

Zur Überprüfung der Funktionalität des codierten Enzyms in einem Mikroorganismus, wird das 1467 bp BamHI/SalI-Fragment aus pBS-Cer1 in den BamHI/XhoI geschnittenen Expressionsvector pYES2 (Invitrogen, Groningen, Niederlande) ligiert und Hefe nach Standardprotokollen mit dem neu entstandenen Plasmid pYES2-Cer1 transformiert (siehe Transformationsprotokoll Invitrogen, Groningen, Niederlande). Erhaltene Kolonien werden auf Raffinosehaltigen Medium angezogen und mittels Galaktose die Genexpression der D6-Acetylenase/Desaturase induziert (siehe unten).

### Beispiel 9:

### Lipidanalyse von transformierten Hefen

Hefen können neben den eigenen Fettsäuren (16:0, 16:1, 18:0 und 18:1) auch exogene Fettsäuren in ihre Membranlipide inkorporieren. Um die Substratspezifität der jeweils exprimierten Desaturase zu testen, wird dem CM - 2 % Raffinose-Medium zur Solubilisierung exogener Fettsäuren 1 % Tergitol NP-40 (w/v, Sigma) und 0,003 % der entsprechenden Fettsäure (Stammlösung: 0,3 % bzw. 3 % Fettsäure in 5 % Tergitol NP-40, w/v) vor der Inokulation zugegeben. Die Vorkultur erfolgte durch Inokulation von 3 ml CM- 2 % Raffinose-Medium / 1 % Tergitol NP-40 mit einer transgenen Hefekolonie und anschließender Inkubation für 2 d bei 30°C im Roller bis zu einer optischen Dichte bei 600 nm (OD₆₀₀) von 4,0 bis 4,3. Für die Hauptkultur werden 10 ml CM- 2 % Raffinose/1 % Tergitol NP-40-Medium ± 0,003 % Fettsäure mit einem Aliquot der Vorkultur (200 fache Verdünnung) ad OD₆₀₀ 0,02 angeimpft und 24 h bei 30°C, 250 rpm im Schüttler inkubiert. Die Induktion der Testkulturen erfolgte in der logarithmischen Wachstumsphase (OD₆₀₀ 0,5 bis 0,6) durch Zugabe von Galaktose ad 1,8 %. Die Ernte der induzierten Zellen erfolgte nach weiteren 24 h aeroben Wachstums bei 30°C bei einer OD₆₀₀ von 4,0 bis 4,3.

Die induzierten Hefezellen werden durch 10 min Zentrifugation bei 2000 g geerntet, in 3 ml Aqua dest. resuspendiert, 10 min bei 100°C abgekocht und nach dem Abkühlen auf Eis erneut sedimentiert. Das Zellsediment wird mit 1 N methanolischer Schwefelsäure und 2 % Dimethoxypropan 1h bei 90°C hydrolysiert und die Lipide transmethyliert. Die resultierenden Fettsäuremethylester (FAME) werden in Petrolether extrahiert. Die extrahierten FAME werden durch Gasflüssigkeitschromatographie mit einer Kapillarsäule (Chrompack, WCOT Fused Silica, CP-Wax-52 CB, 25 m, 0,32 mm) und einem Temperaturgradienten von 170°C auf 240°C in 20 min und 5 min bei 240°C analysiert. Die Identität der Mono-, Di-, Tri- und Tetraensäuremethylester wird durch Vergleich mit entsprechenden FAME-Standards (Sigma) bestätigt. Für die Tri- und Tetraynsäuren sind keine Referenzsubstanzen erhältlich. Ihre Identität und die Position der Dreifachbindung wird durch geeignete chemische Derivatisierung der FAME-Gemische z.B. zu 4,4-Dimethoxyoxazolin-Derivaten (Christie, 1998) mittels GC-MS von analysiert. Die GC-Analysen der Fettsäuremethylester aus den transgenen Hefen, die mit dem Leervektor pYES2, mit pYES2-Cer1 (Δ6-Acetylenase) transformiert werden, ist in Tab. 1 dargestellt. Die transgenen Hefezellen werden ohne exogenen Fettsäuren oder nach Zugabe von Linolsäure (18:2), γ-Linolensäure (γ-18:3), α-Linolensäure (α-18:3) oder ω3-Octadecatetraensäure (18:4) analysiert.

Tabelle 1 gibt die GC-Analysen der Fettsäuremethylester aus transgenen Hefen, die mit dem Leervektor pYES2, der Δ6-Acetylenase (Cer1/pYES2) und der Δ6-Desaturase (Cer3/pYES2) transformiert wurden, wieder. Die transgenen Hefezellen wurden ohne exogenen Fettsäuren ( - ) oder nach Zugabe von Linolsäure (18:2), γ-Linolensäure (γ-18:3), α-Linolensäure (α-18:3) oder ω3-Octadecatetraensäure (18:4) analysiert. Fettsäurezusammensetzung in [mol %] der Gesamtfettsäuren, wobei die Inkorporation der gefütterten Fettsäuren (schwarzer Fettdruck), die Desaturierungsprodukte (in roter Farbe) und die Summe der Desaturierungsprodukte (letzte Zeile) bei den einzelnen Fütterungsversuchen angegeben sind.

### Beispiel 10:

Erzeugung transgener Pflanzen, welche ein Enzym mit D6-Acetylenase/Desaturase-Aktivität überexprimieren.

Für die Transformation von Pflanzen wird ein Transformationsvektor erzeugt, der das BamHI/SalI-Fragment aus pBS-Cer1 in den mit BamHI/SalI gespaltenen Vektor pBin-USP oder in pBinAR ligiert wird. pBin-USP und pBinAR sind Derivate des Plasmides pBin19. pBinAR entstand aus pBin19, indem in pBin19 (Bevan et al. (1980) Nucl. Acids Res. 12, 8711) ein 35S CaMV Promotor als EcoRI-KpnI-Fragment (entsprechend den Nukleotiden 6909-7437 des Cauliflower-Mosaik-Virus (Franck et al. (1980) Cell 21, 285) inseriert wird. Das Polyadenylierungssignal des Gens 3 der T-DNA des Ti-Plasmides pTiACH5 (Gielen et al., (1984) EMBO J. 3, 835), Nukleotide 11749-11939 wird als PvuII-HindIII-Fragment isoliert und nach Addition von SphI-Linkern an die PvuII-Schnittstelle zwischen die SpHI-HindIII Schnittstelle des Vektors kloniert. Es entstand das Plasmid pBinAR (Höfgen und Willmitzer (1990) Plant Science 66, 221-230), wobei durch Umklonierung aus pBluescript mehrere Restriktionschnittstellen zwischen Promotor und Terminator zur Verfügung stehen. Der USP-Promotor entspricht den Nukleotiden 1-684 (Genbank Accession X56240), wobei ein Teil der nichtcodierenden Region des USP-Gens im Promotor enthalten ist.

Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert. (Primersequenz: 5'-GTCGACCCGCGGACTAGTGGGCCCTCTAGACCCGGGGGATCCGGATCTGCTGGCTATGAA-3'). Das PCR-Fragment wurde mit EcoRI/SalI nachgeschnitten und in den Vektor pBinAR eingesetzt. Es entsteht das Plasmid mit der Bezeichnung pBinUSP.

Das Konstrukt wird zur Transformation von Arabidopsis thaliana und Rapspflanzen eingesetzt.

Regenerierte Sprosse werden auf 2MS-Medium mit Kanamycin und Claforan erhalten, nach Bewurzelung in Erde überführt und nach Kultivierung für zwei Wochen in einer Klimakammer oder im Gewächshaus angezogen, zur Blüte gebracht, reife Samen geerntet und auf D6-Acetylenase/Desaturase -Expression mittels Lipidanalysen untersucht. Linien mit erhöhten Gehalten an Acetylenfettsäuren oder Doppelbindungen an der delta-6-position werden identifiziert. Es läßt sich in den stabil transformierten transgenen Linien, die das Transgen funktionell exprimieren, ein erhöhter Gehalt von Acetylenfettsäuren und Doppelbindungen an der delta-6-position im Vergleich zu untransformierten Kontrollpflanzen feststellen.

### Beispiel 11:

### Lipidextraktion aus Samen

Die Analyse von Lipiden aus Pflanzensamen verläuft analog der Analyse von Hefelipiden. Jedoch wird Pflanzenmaterial zunächst mechanisch durch Mörsern homogenisert, um es einer Extraktion zugänglich zu machen.

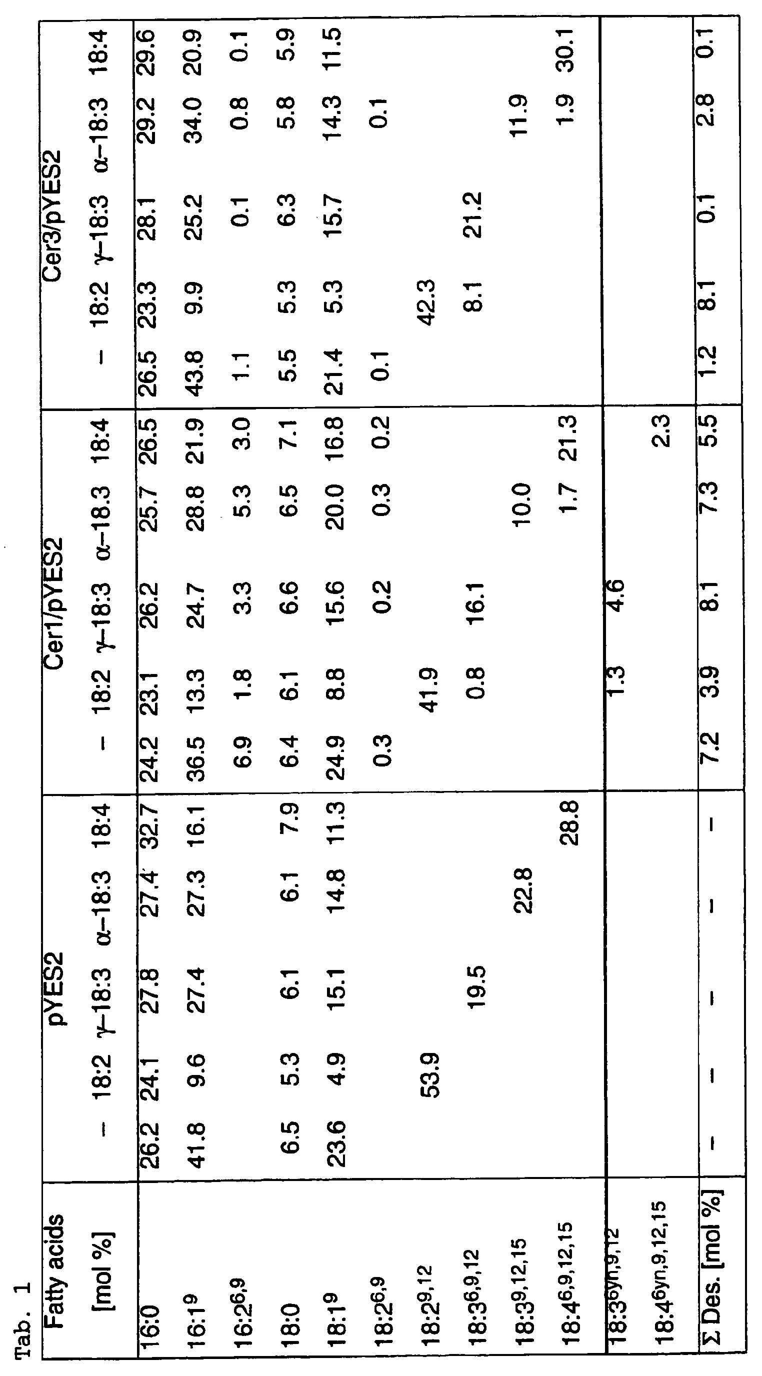

## Patentansprüche

1. Isolierte Nukleinsäure, die für ein Polypeptid mit Δ6-Acetylenase- und Δ-6-Desaturaseaktivität codiert, ausgewählt aus der Gruppe:
a) einer Nukleinsäure mit der in SEQ ID NO: 1 oder SEQ ID NO: 3 dargestellten Sequenz,
b) Nukleinsäuren, die sich als Ergebnis des degenerierten genetischen Codes von der in SEQ ID NO: 1 oder SEQ ID NO: 3 dargestellten Nukleinsäure ableiten,
c) Nukleinsäuren, die für Polypeptide codieren die zu den Polypeptiden mit den in SEQ ID NO: 2 oder SEQ ID NO: 4 dargestellten Aminosäuresequenzen mindestens 75 % Identität aufweisen, ohne daß die enzymatische Wirkung der Polypeptide wesentlich reduziert ist.

2. Protein codiert durch eine Nukleinsäure gemäß Anspruch 1.

3. Protein nach Anspruch 2, codiert durch die Nukleinsäuren mit den in SEQ ID NO: 1 oder SEQ ID NO: 3 dargestellten Sequenz.

4. Expressionskassette enthaltend eine Nukleinsäure gemäß Anspruch 1, wobei die Nukleinsäure mit einem oder mehreren Regulationssignalen verknüpft ist.

5. Vektor enthaltend eine Nukleinsäure gemäß Anspruch 1 oder eine Expressionskassette gemäß Anspruch 4.

6. Transgener nicht-humaner Organismus, in den mindestens eine Nukleinsäure gemäß Anspruch 1 oder mindestens ein Expressionskassette gemäß Anspruch 4 oder mindestens einen Vektor gemäß Anspruch 5 eingebracht wurde.

7. Transgener nicht-humaner Organismus nach Anspruch 6, wobei es sich bei dem Organismus um eine Pflanze, einen Mikroorganismus oder ein Tier handelt.

8. Verfahren zur Herstellung von ungesättigten Fettsäuren **dadurch gekennzeichnet, daß** man mindestens eine Nukleinsäure gemäß Anspruch 1 oder mindestens eine Expressions-kassette gemäß Anspruch 4 in einen Öl produzierenden nicht-humanen Organismus bringt, diesen Organismus anzieht und daß in dem Organismus enthaltene Öl isoliert und die im Öl enthaltenden Fettsäuren freisetzt.

9. Verfahren zur Herstellung von Triglyceriden mit einem erhöhten Gehalt an ungesättigten Fettsäuren, **dadurch gekennzeichnet, daß** man mindestens eine Nukleinsäure gemäß Anspruch 1 oder mindestens eine Expressionskassette gemäß Anspruch 4 in einen Ö1 produzierenden nicht-humanen Organismus bringt, diesen Organismus anzieht und daß in dem Organismus enthaltene Öl isoliert.

10. Verfahren nach Anspruch 8 oder 9, **dadurch gekennzeichnet, daß** die ungesättigten Fettsäuren einen erhöhten Gehalt an ungesättigten Fettsäuren mit einer Dreifachbindung oder mit einer Doppelbindung in Δ-6-Position oder einer Dreifachbindung und Doppelbindung in Δ-6-Position aufweisen.

11. Verfahren nach den Ansprüchen 8 bis 10, **dadurch gekennzeichnet, daß** es sich bei dem Organismus um eine Pflanze oder einen Mikroorganismus handelt.

12. Verfahren nach Anspruch 11, **dadurch gekennzeichnet, daß** es sich bei dem Organismus um eine Kulturpflanze handelt.

13. Verfahren nach Anspruch 8 oder 9, **dadurch gekennzeichnet, daß** die im Verfahren hergestellten ungesättigten Fettsäuren oder Triglyceride mit einem erhöhten Gehalt an ungesättigten Fettsäuren Nahrungsmitteln, Tierfutter, Kosmetika oder Pharmazeutika zusetzt werden.

14. Verfahren zur Herstellung von Triglyceriden mit einem erhöhten Gehalt an ungesättigten Fettsäuren, indem man Triglyceride mit gesättigten oder ungesättigten oder gesättigten und ungesättigten Fettsäuren mit mindestens einem der Proteine gemäß Anspruch 2 inkubiert.

15. Verfahren nach Anspruch 14, **dadurch gekennzeichnet, daß** die Triglyceride in Gegenwart einer Verbindung hergestellt werden, die Reduktionsäquivalente aufnehmen oder abgeben können.

16. Verfahren nach Anspruch 14 oder 15, **dadurch gekennzeichnet, daß** die Fettsäuren aus den Triglyceriden freigesetzt werden.

17. Verwendung einer Nukleinsäure gemäß Anspruch 1 oder eine Expressionskassette gemäß Anspruch 4 zur Herstellung von transgenen Pflanzen.

## Claims

1. An isolated nucleic acid which codes for a polypeptide having Δ6-acetylenase and Δ6-desaturase activity, selected from the group:
a) of a nucleic acid having the sequence depicted in SEQ ID NO: 1 or SEQ ID NO: 3,
b) nucleic acids which, as a result of the degeneracy of the genetic code, are derived from the nucleic acid depicted in SEQ ID NO: 1 or SEQ ID NO: 3,
c) nucleic acids which code for polypeptides having at least 75% identity with the polypeptides having the amino acid sequences depicted in SEQ ID NO: 2 or SEQ ID NO: 4, with a negligible reduction in the enzymatic action of the polypeptides.

2. A protein encoded by the nucleic acid according to claim 1.

3. The protein according to claim 2, encoded by the nucleic acids having the sequence depicted in SEQ ID NO: 1 or SEQ ID NO: 3.

4. An expression cassette comprising the nucleic acid according to claim 1, where the nucleic acid is linked to one or more regulatory signals.

5. A vector comprising the nucleic acid according to claim 1 or the expression cassette according to claim 4.

6. A transgenic non-human organism into which at least one nucleic acid according to claim 1 or at least one expression cassette according to claim 4 or at least one vector according to claim 5 has been introduced.

7. The transgenic non-human organism according to claim 6, where the organism is a plant, a microorganism or an animal.

8. A process for preparing unsaturated fatty acids, which comprises introducing at least one nucleic acid according to claim 1 or at least one expression cassette according to claim 4 into an oil-producing non-human organism, culturing this organism and isolating the oil contained in the organism, and liberating the fatty acids contained in the oil.

9. A process for preparing triglycerides with an increased content of unsaturated fatty acids, which comprises introducing at least one nucleic acid according to claim 1 or at least one expression cassette according to claim 4 into an oil-producing non-human organism, culturing this organism and isolating the oil contained in the organism.

10. The process according to claim 8 or 9, wherein the unsaturated fatty acids have an increased content of unsaturated fatty acids with a triple bond or with a double bond in position 6 or a triple bond and double bond in position 6.

11. The process according to any of claims 8 to 10, wherein the organism is a plant or a microorganism.

12. The process according to claim 11, wherein the organism is a crop plant

13. The process according to claim 8 or 9, wherein the unsaturated fatty acids or triglycerides with an increased content of unsaturated fatty acids prepared in the process are added to human foods, animal feed, cosmetics or pharmaceuticals.

14. A process for preparing triglycerides with an increased content of unsaturated fatty acids by incubating triglycerides with saturated or unsaturated or saturated and unsaturated fatty acids with at least one of the proteins according to claim 2.

15. The process according to claim 14, wherein the triglycerides are prepared in the presence of a compound which is able to take up or release reducing equivalents.

16. The process according to claim 14 or 15, wherein the fatty acids are liberated from the triglycerides.

17. The use of the nucleic acid according to claim 1 or the expression cassette according to claim 4 for producing transgenic plants.

## Revendications

1. Acide nucléique isolé, qui code pour un polypeptide ayant une activité de Δ-6-acétylénase et de Δ-6-désaturase, choisi parmi le groupe :
a) d'un acide nucléique ayant la séquence représentée dans SEQ ID NO: 1 ou SEQ ID NO: 3,
b) d'acides nucléiques qui, comme résultat du code génétique dégénéré, dérivent de l'acide nucléique représenté dans SEQ ID NO: 1 ou SEQ ID NO: 3,
c) d'acides nucléiques qui codent pour des polypeptides qui présentent une identité d'au moins 75 % avec les polypeptides ayant les séquences d'acides aminés représentées dans SEQ ID NO: 2 ou SEQ ID NO: 4, sans que l'action enzymatique des polypeptides ne soit sensiblement réduite.

2. Protéine codée par un acide nucléique suivant la revendication 1.

3. Protéine suivant la revendication 2, codée par les acides nucléiques comportant la séquence représentée dans SEQ ID NO: 1 ou SEQ ID NO: 3.

4. Cassette d'expression contenant un acide nucléique suivant la revendication 1, l'acide nucléique étant relié à un ou plusieurs signaux de régulation.

5. Vecteur contenant un acide nucléique suivant la revendication 1 ou une cassette d'expression suivant la revendication 4.

6. Organisme non humain transgénique, dans lequel au moins un acide nucléique suivant la revendication 1 ou au moins une cassette d'expression suivant la revendication 4 ou au moins un vecteur suivant la revendication 5 a été introduit.

7. Organisme non humain transgénique suivant la revendication 6, dans lequel, pour ce qui concerne l'organisme, il s'agit d'une plante, d'un micro-organisme ou d'un animal.

8. Procédé de préparation d'acides gras insaturés, **caractérisé en ce qu'**on amène au moins un acide nucléique suivant la revendication 1 ou au moins une cassette d'expression suivant la revendication 4 dans un organisme non humain produisant de l'huile, on cultive cet organisme et on isole l'huile contenue dans l'organisme et on libère les acides gras contenus dans l'huile.

9. Procédé de préparation de triglycérides ayant une teneur élevée en acides gras insaturés, **caractérisé en ce qu'**on amène au moins un acide nucléique suivant la revendication 1 ou au moins une cassette d'expression suivant la revendication 4 dans un organisme non humain produisant de l'huile, on cultive cet organisme et on isole l'huile contenue dans l'organisme.

10. Procédé suivant la revendication 8 ou 9, **caractérisé en ce que** les acides gras insaturés présentent une teneur élevée en acides gras insaturés comportant une triple liaison ou une double liaison en position Δ-6 ou une triple liaison et une double liaison en position Δ-6.

11. Procédé suivant les revendications 8 à 10, **caractérisé en ce que**, pour ce qui concerne l'organisme, il s'agit d'une plante ou d'un micro-organisme.

12. Procédé suivant la revendication 11, **caractérisé en ce que**, pour ce qui concerne l'organisme, il s'agit d'une plante de culture.

13. Procédé suivant la revendication 8 ou 9, **caractérisé en ce que** les acides gras insaturés ou triglycérides comportant une teneur élevée en acides gras insaturés, préparés dans le procédé, sont ajoutés à des produits nutritifs, des aliments pour animaux, des cosmétiques ou des produits pharmaceutiques.

14. Procédé de préparation de triglycérides comportant une teneur élevée en acides gras insaturés, dans lequel on met à incuber des triglycérides avec des acides gras saturés ou insaturés ou saturés et insaturés avec au moins une des protéines selon la revendication 2.

15. Procédé suivant la revendication 14, **caractérisé en ce que** les triglycérides sont préparés en présence d'un composé qui peut capter ou céder des équivalents de réduction.

16. Procédé suivant la revendication 14 ou 15, **caractérisé en ce que** les acides gras sont libérés des triglycérides.

17. Utilisation d'un acide nucléique suivant la revendication 1 ou d'une cassette d'expression suivant la revendication 4, pour la préparation de plantes transgéniques.


### SEQUENZPROTOKOLL

<110> BASF Aktiengesellschaft
<120> D6-Acetylenase und D6-Desaturase aus Ceratodon purpureus
<130> 99 1388
<140>
   <141>
<150> 19925718.3
   <151> 1999-06-07
<160> 12
<170> PatentIn Vers. 2.0
<210> 1
   <211> 2040
   <212> DNA
   <213> Ceratodon purpureus
<220>
   <221> CDS
   <222> (176)..(1627)
<400> 1
   ctcaggcagg tctcagttga tgagacgctg agttctgaat cctttgagct gtgtcaggct 60
   cggcacttgt gggatggtga aggagtgatc gatcaggagt gcaggagctg cattagtttc 120
   tcagggtcga tcaggttatt ctgaaaaagg ctgcgtctgt gagcagtttg caaaa atg  178
                                                                Met
                                                                 1
   gcc ctc gtt acc gac ttt ctg aac ttt ctg ggc acg aca tgg agc aag   226
   Ala Leu Val Thr Asp Phe Leu Asn Phe Leu Gly Thr Thr Trp Ser Lys
                 5                  10                  15
   tac agc gtg tac acc cat agc tat gct gga aac tat ggg cct act ttg   274
   Tyr Ser Val Tyr Thr His Ser Tyr Ala Gly Asn Tyr Gly Pro Thr Leu
            20                  25                  30
   aag cac gcc aaa aag gtt tct gct caa ggt aaa act gcg gga cag aca   322
   Lys His Ala Lys Lys Val Ser Ala Gln Gly Lys Thr Ala Gly Gln Thr
        35                  40                  45
   ctg aga cag aga tcg gtg cag gac aaa aag cca ggc act tac tct ctg   370
   Leu Arg Gln Arg Ser Val Gln Asp Lys Lys Pro Gly Thr Tyr Ser Leu
               50                  55                  60                  65
   gcc gat gtt gct tct cac gac agg cct gga gac tgc tgg atg atc gtc    418
   Ala Asp Val Ala Ser His Asp Arg Pro Gly Asp Cys Trp Met Ile Val
                       70                  75                  80
   aaa gag aag gtg tat gat att agc cgt ttt gcg gac gac cac cct gga    466
   Lys Glu Lys Val Tyr Asp Ile Ser Arg Phe Ala Asp Asp His Pro Gly
                   85                  90                  95
   ggg acg gta att agc acc tac ttt ggg cgg gat ggc aca gac gtt ttc    514
   Gly Thr Val Ile Ser Thr Tyr Phe Gly Arg Asp Gly Thr Asp Val Phe
               100                 105                 110
   gca aca ttc cat cca cct gcc gca tgg aag caa ctc aat gac tac tac    562
   Ala Thr Phe His Pro Pro Ala Ala Trp Lys Gln Leu Asn Asp Tyr Tyr
           115                 120                 125
   att gga gac ctt gct agg gaa gag ccc ctt gat gaa ttg ctt aaa gac    610
   Ile Gly Asp Leu Ala Arg Glu Glu Pro Leu Asp Glu Leu Leu Lys Asp
   130                 135                 140                 145
   tac aga gat atg aga gcc gag ttt gtt aga gaa ggg ctt ttc aag agt    658
   Tyr Arg Asp Met Arg Ala Glu Phe Val Arg Glu Gly Leu Phe Lys Ser
                   150                 155                 160
   tcc aag gcc tgg ttc ctg ctt cag act ctg att aat gca gct ctc ttt    706
   Ser Lys Ala Trp Phe Leu Leu Gln Thr Leu Ile Asn Ala Ala Leu Phe
               165                 170                 175
   gct gcg agc att gcg act atc tgt tac gac aag agt tac tgg gct att    754
   Ala Ala Ser Ile Ala Thr Ile Cys Tyr Asp Lys Ser Tyr Trp Ala Ile
           180                 185                 190
   gtg ctg tca gcc agt ttg atg ggt ctc ttc gtc caa cag tgt gga tgg    802
   Val Leu Ser Ala Ser Leu Met Gly Leu Phe Val Gln Gln Cys Gly Trp
       195                 200                 205
   ctt gcc cat gat ttc ctt cat caa cag gtc ttt gag aac cgt acc gcg    850
   Leu Ala His Asp Phe Leu His Gln Gln Val Phe Glu Asn Arg Thr Ala
   210                 215                 220                 225
   aac tcc ttc ttt ggc tat ttg ttc ggc aat tgc gtg ctt ggc ttt agt    898
   Asn Ser Phe Phe Gly Tyr Leu Phe Gly Asn Cys Val Leu Gly Phe Ser
                   230                 235                 240
   gta tca tgg tgg agg acg aag cac aac att cat cat act gct ccg aat    946
   Val Ser Trp Trp Arg Thr Lys His Asn Ile His His Thr Ala Pro Asn
               245                 250                 255
   gag tgc gac gaa cag tac aca cct cta gac gaa gac att gat act ctc    994
   Glu Cys Asp Glu Gln Tyr Thr Pro Leu Asp Glu Asp Ile Asp Thr Leu
           260                 265                 270
   ccc atc att gcc tgg agc aag gaa att ttg gcc acc gtt gag agc aag    1042
   Pro Ile Ile Ala Trp Ser Lys Glu Ile Leu Ala Thr Val Glu Ser Lys
       275                 280                 285
   aga att ttg cga gtg ctt caa tat cag cac tac atg att ctg cct cta    1090
   Arg Ile Leu Arg Val Leu Gln Tyr Gln His Tyr Met Ile Leu Pro Leu
   290                 295                 300                 305
   ttg ttc atg gcc cgg tac agt tgg act ttt gga agt ttg ctc ttc aca    1138
   Leu Phe Met Ala Arg Tyr Ser Trp Thr Phe Gly Ser Leu Leu Phe Thr
                   310                 315                 320
   ttc aat cct gat ttg agc acg acc aag gga ttg ata gag aag gga aca    1186
   Phe Asn Pro Asp Leu Ser Thr Thr Lys Gly Leu Ile Glu Lys Gly Thr
               325                 330                 335
   gtt gct ttt cac tac gcc tgg ttc agt tgg gct gcg ttc cat att ttg    1234
   Val Ala Phe His Tyr Ala Trp Phe Ser Trp Ala Ala Phe His Ile Leu
           340                 345                 350
   ccg ggt gtc gct aag cct ctt gcg tgg atg gta gca act gag ctt gtg    1282
   Pro Gly Val Ala Lys Pro Leu Ala Trp Met Val Ala Thr Glu Leu Val
       355                 360                 365
   gcc ggt ttg ttg ttg gga ttc gtg ttt acg ttg agt cac aat gga aag    1330
   Ala Gly Leu Leu Leu Gly Phe Val Phe Thr Leu Ser His Asn Gly Lys
   370                 375                 380                 385
   gag gtt tac aat gaa tcg aag gac ttc gtg aga gcc cag gtt att acc    1378
   Glu Val Tyr Asn Glu Ser Lys Asp Phe Val Arg Ala Gln Val Ile Thr
                   390                 395                 400
   acc cgt aac acc aag cga ggc tgg ttc aac gat tgg ttc act ggg gga    1426
   Thr Arg Asn Thr Lys Arg Gly Trp Phe Asn Asp Trp Phe Thr Gly Gly
               405                 410                 415
   ctc gac acc cag att gag cat cac ctg ttt cca aca atg ccc agg cac    1474
   Leu Asp Thr Gln Ile Glu His His Leu Phe Pro Thr Met Pro Arg His
           420                 425                 430
   aac tac ccc aag atc gca cct cag gtc gag gct ctt tgc aag aag cac    1522
   Asn Tyr Pro Lys Ile Ala Pro Gln Val Glu Ala Leu Cys Lys Lys His
       435                 440                 445
   ggc ctc gag tac gat aat gtc tcc gtc gtt ggt gcc tct gtc gcg gtt    1570
   Gly Leu Glu Tyr Asp Asn Val Ser Val Val Gly Ala Ser Val Ala Val
   450                 455                 460                 465
   gtg aag gcg ctc aag gaa att gct gat gaa gcg tca att cgg ctt cac    1618
   Val Lys Ala Leu Lys Glu Ile Ala Asp Glu Ala Ser Ile Arg Leu His
                   470                 475                 480
   gct cac taa gaaatcgtcg aactttgact attcattttt ttcgcctggc            1667
   Ala His
   tacctcaaat gttcgggagc aggtgcttgg cagtgtgttc aaccggagcg cactgaaaat 1727
   gtgcagaatc catttccaga aattaccatt cctagctaaa tcttcttttt accaggtcgg 1787
   atatatgaaa cttttttgat gcaacaagta gcattcaatt gaagacattg ttcgagatat 1847
   aattcgcagt gtttctattc agcgggcata cgtactagtc catatcggcg gttgccgaga 1907
   gtttacatta ttagttggca caacgagtag atctagtgta aatttctatt tccgcatgta 1967
   atattactct gaatatatac cgttatctat tttcctaaaa aaaaaaaaaa aaaaaaaaaa 2027
   aaaaaaaaaa aaa                                                     2040
<210> 2
   <211> 483
   <212> PRT
   <213> Ceratodon purpureus
<400> 2
   Met Ala Leu Val Thr Asp Phe Leu Asn Phe Leu Gly Thr Thr Trp Ser
   1 5 10 15
   Lys Tyr Ser Val Tyr Thr His Ser Tyr Ala Gly Asn Tyr Gly Pro Thr
   20 25 30
   Leu Lys His Ala Lys Lys Val Ser Ala Gln Gly Lys Thr Ala Gly Gln
   35 40 45
   Thr Leu Arg Gln Arg Ser Val Gln Asp Lys Lys Pro Gly Thr Tyr Ser
   50 55 60
   Leu Ala Asp Val Ala Ser His Asp Arg Pro Gly Asp Cys Trp Met Ile
   65 70 75 80
   Val Lys Glu Lys Val Tyr Asp Ile Ser Arg Phe Ala Asp Asp His Pro
   85 90 95
   Gly Gly Thr Val Ile Ser Thr Tyr Phe Gly Arg Asp Gly Thr Asp Val
   100 105 110
   Phe Ala Thr Phe His Pro Pro Ala Ala Trp Lys Gln Leu Asn Asp Tyr
   115 120 125
   Tyr Ile Gly Asp Leu Ala Arg Glu Glu Pro Leu Asp Glu Leu Leu Lys
       130                 135                 140
   Asp Tyr Arg Asp Met Arg Ala Glu Phe Val Arg Glu Gly Leu Phe Lys
   145                 150                 155                 160
   Ser Ser Lys Ala Trp Phe Leu Leu Gln Thr Leu Ile Asn Ala Ala Leu
                   165                 170                 175
   Phe Ala Ala Ser Ile Ala Thr Ile Cys Tyr Asp Lys Ser Tyr Trp Ala
               180                 185                 190
   Ile Val Leu Ser Ala Ser Leu Met Gly Leu Phe Val Gln Gln Cys Gly
           195                 200                 205
   Trp Leu Ala His Asp Phe Leu His Gln Gln Val Phe Glu Asn Arg Thr
       210                 215                 220
   Ala Asn Ser Phe Phe Gly Tyr Leu Phe Gly Asn Cys Val Leu Gly Phe
   225                 230                 235                 240
   Ser Val Ser Trp Trp Arg Thr Lys His Asn Ile His His Thr Ala Pro
                   245                 250                 255
   Asn Glu Cys Asp Glu Gln Tyr Thr Pro Leu Asp Glu Asp Ile Asp Thr
               260                 265                 270
   Leu Pro Ile Ile Ala Trp Ser Lys Glu Ile Leu Ala Thr Val Glu Ser
           275                 280                 285
   Lys Arg Ile Leu Arg Val Leu Gln Tyr Gln His Tyr Met Ile Leu Pro
       290                 295                 300
   Leu Leu Phe Met Ala Arg Tyr Ser Trp Thr Phe Gly Ser Leu Leu Phe
   305                 310                 315                 320
   Thr Phe Asn Pro Asp Leu Ser Thr Thr Lys Gly Leu Ile Glu Lys Gly
                   325                 330                 335
   Thr Val Ala Phe His Tyr Ala Trp Phe Ser Trp Ala Ala Phe His Ile
               340                 345                 350
   Leu Pro Gly Val Ala Lys Pro Leu Ala Trp Met Val Ala Thr Glu Leu
           355                 360                 365
   Val Ala Gly Leu Leu Leu Gly Phe Val Phe Thr Leu Ser His Asn Gly
       370                 375                 380
   Lys Glu Val Tyr Asn Glu Ser Lys Asp Phe Val Arg Ala Gln Val Ile
   385                 390                 395                 400
   Thr Thr Arg Asn Thr Lys Arg Gly Trp Phe Asn Asp Trp Phe Thr Gly
                   405                     410                 415
   Gly Leu Asp Thr Gln Ile Glu His His Leu Phe Pro Thr Met Pro Arg
               420                     425                 430
   His Asn Tyr Pro Lys Ile Ala Pro Gln Val Glu Ala Leu Cys Lys Lys
           435                     440                 445
   His Gly Leu Glu Tyr Asp Asn Val Ser Val Val Gly Ala Ser Val Ala
       450                     455                 460
   Val Val Lys Ala Leu Lys Glu Ile Ala Asp Glu Ala Ser Ile Arg Leu
   465                     470                 475                 480
   His Ala His
<210> 3
   <211> 1467
   <212> DNA
   <213> Ceratodon purpureus
<220>
   <221> CDS
   <222> (10)..(1461)
<400> 3
   ggatccaaa atg gcc ctc gtt acc gac ttt ctg aac ttt ctg ggc acg aca 51
             Met Ala Leu Val Thr Asp Phe Leu Asn Phe Leu Gly Thr Thr
              1               5                  10
   tgg agc aag tac agc gtg tac acc cat agc tat gct gga aac tat ggg   99
   Trp Ser Lys Tyr Ser Val Tyr Thr His Ser Tyr Ala Gly Asn Tyr Gly
    15                  20                  25                  30
   cct act ttg aag cac gcc aaa aag gtt tct gct caa ggt aaa act gcg   147
   Pro Thr Leu Lys His Ala Lys Lys Val Ser Ala Gln Gly Lys Thr Ala
                    35                  40                  45
   gga cag aca ctg aga cag aga tcg gtg cag gac aaa aag cca ggc act   195
   Gly Gln Thr Leu Arg Gln Arg Ser Val Gln Asp Lys Lys Pro Gly Thr
                50                  55                  60
   tac tct ctg gcc gat gtt gct tct cac gac agg cct gga gac tgc tgg   243
   Tyr Ser Leu Ala Asp Val Ala Ser His Asp Arg Pro Gly Asp Cys Trp
            65                  70                  75
   atg atc gtc aaa gag aag gtg tat gat att agc cgt ttt gcg gac gac   291
   Met Ile Val Lys Glu Lys Val Tyr Asp Ile Ser Arg Phe Ala Asp Asp
           80                  85                  90
   cac cct gga ggg acg gta att agc acc tac ttt ggg cgg gat ggc aca    339
   His Pro Gly Gly Thr Val Ile Ser Thr Tyr Phe Gly Arg Asp Gly Thr
    95                 100                 105                 110
   gac gtt ttc gca aca ttc cat cca cct gcc gca tgg aag caa ctc aat    387
   Asp Val Phe Ala Thr Phe His Pro Pro Ala Ala Trp Lys Gln Leu Asn
                   115                 120                 125
   gac tac tac att gga gac ctt gct agg gaa gag ccc ctt gat gaa ttg    435
   Asp Tyr Tyr Ile Gly Asp Leu Ala Arg Glu Glu Pro Leu Asp Glu Leu
               130                 135                 140
   ctt aaa gac tac aga gat atg aga gcc gag ttt gtt aga gaa ggg ctt    483
   Leu Lys Asp Tyr Arg Asp Met Arg Ala Glu Phe Val Arg Glu Gly Leu
           145                 150                 155
   ttc aag agt tcc aag gcc tgg ttc ctg ctt cag act ctg att aat gca    531
   Phe Lys Ser Ser Lys Ala Trp Phe Leu Leu Gln Thr Leu Ile Asn Ala
       160                 165                 170
   gct ctc ttt gct gcg agc att gcg act atc tgt tac gac aag agt tac    579
   Ala Leu Phe Ala Ala Ser Ile Ala Thr Ile Cys Tyr Asp Lys Ser Tyr
   175                 180                 185                 190
   tgg gct att gtg ctg tca gcc agt ttg atg ggt ctc ttc gtc caa cag    627
   Trp Ala Ile Val Leu Ser Ala Ser Leu Met Gly Leu Phe Val Gln Gln
                   195                 200                 205
   tgt gga tgg ctt gcc cat gat ttc ctt cat caa cag gtc ttt gag aac    675
   Cys Gly Trp Leu Ala His Asp Phe Leu His Gln Gln Val Phe Glu Asn
               210                 215                 220
   cgt acc gcg aac tcc ttc ttt ggc tat ttg ttc ggc aat tgc gtg ctt    723
   Arg Thr Ala Asn Ser Phe Phe Gly Tyr Leu Phe Gly Asn Cys Val Leu
           225                 230                 235
   ggc ttt agt gta tca tgg tgg agg acg aag cac aac att cat cat act    771
   Gly Phe Ser Val Ser Trp Trp Arg Thr Lys His Asn Ile His His Thr
       240                 245                 250
   gct ccg aat gag tgc gac gaa cag tac aca cct cta gac gaa gac att    819
   Ala Pro Asn Glu Cys Asp Glu Gln Tyr Thr Pro Leu Asp Glu Asp Ile
   255                 260                 265                 270
   gat act ctc ccc atc att gcc tgg agc aag gaa att ttg gcc acc gtt    867
   Asp Thr Leu Pro Ile Ile Ala Trp Ser Lys Glu Ile Leu Ala Thr Val
                   275                 280                 285
   gag agc aag aga att ttg cga gtg ctt caa tat cag cac tac atg att    915
   Glu Ser Lys Arg Ile Leu Arg Val Leu Gln Tyr Gln His Tyr Met Ile
               290                 295                 300
   ctg cct cta ttg ttc atg gcc cgg tac agt tgg act ttt gga agt ttg      963
   Leu Pro Leu Leu Phe Met Ala Arg Tyr Ser Trp Thr Phe Gly Ser Leu
           305                 310                 315
   ctc ttc aca ttc aat cct gat ttg agc acg acc aag gga ttg ata gag     1011
   Leu Phe Thr Phe Asn Pro Asp Leu Ser Thr Thr Lys Gly Leu Ile Glu
       320                 325                 330
   aag gga aca gtt gct ttt cac tac gcc tgg ttc agt tgg gct gcg ttc     1059
   Lys Gly Thr Val Ala Phe His Tyr Ala Trp Phe Ser Trp Ala Ala Phe
   335                 340                 345                 350
   cat att ttg ccg ggt gtc gct aag cct ctt gcg tgg atg gta gca act     1107
   His Ile Leu Pro Gly Val Ala Lys Pro Leu Ala Trp Met Val Ala Thr
                   355                 360                 365
   gag ctt gtg gcc ggt ttg ttg ttg gga ttc gtg ttt acg ttg agt cac     1155
   Glu Leu Val Ala Gly Leu Leu Leu Gly Phe Val Phe Thr Leu Ser His
               370                 375                 380
   aat gga aag gag gtt tac aat gaa tcg aag gac ttc gtg aga gcc cag     1203
   Asn Gly Lys Glu Val Tyr Asn Glu Ser Lys Asp Phe Val Arg Ala Gln
           385                 390                 395
   gtt att acc acc cgt aac acc aag cga ggc tgg ttc aac gat tgg ttc     1251
   Val Ile Thr Thr Arg Asn Thr Lys Arg Gly Trp Phe Asn Asp Trp Phe
       400                 405                 410
   act ggg gga ctc gac acc cag att gag cat cac ctg ttt cca aca atg     1299
   Thr Gly Gly Leu Asp Thr Gln Ile Glu His His Leu Phe Pro Thr Met
   415                 420                 425                 430
   ccc agg cac aac tac ccc aag atc gca cct cag gtc gag gct ctt tgc     1347
   Pro Arg His Asn Tyr Pro Lys Ile Ala Pro Gln Val Glu Ala Leu Cys
                   435                 440                 445
   aag aag cac ggc ctc gag tac gat aat gtc tcc gtc gtt ggt gcc tct     1395
   Lys Lys His Gly Leu Glu Tyr Asp Asn Val Ser Val Val Gly Ala Ser
               450                 455                 460
   gtc gcg gtt gtg aag gcg ctc aag gaa att gct gat gaa gcg tca att     1443
   Val Ala Val Val Lys Ala Leu Lys Glu Ile Ala Asp Glu Ala Ser Ile
           465                 470                 475
   cgg ctt cac gct cac taa gtcgac                                      1467
   Arg Leu His Ala His
       480
<210> 4
   <211> 483
   <212> PRT
   <213> Ceratodon purpureus
<400> 4
   Met Ala Leu Val Thr Asp Phe Leu Asn Phe Leu Gly Thr Thr Trp Ser
   1               5                   10                  15
   Lys Tyr Ser Val Tyr Thr His Ser Tyr Ala Gly Asn Tyr Gly Pro Thr
               20                  25                  30
   Leu Lys His Ala Lys Lys Val Ser Ala Gln Gly Lys Thr Ala Gly Gln
           35                  40                  45
   Thr Leu Arg Gln Arg Ser Val Gln Asp Lys Lys Pro Gly Thr Tyr Ser
       50                  55                  60
   Leu Ala Asp Val Ala Ser His Asp Arg Pro Gly Asp Cys Trp Met Ile
   65                  70                  75                  80
   Val Lys Glu Lys Val Tyr Asp Ile Ser Arg Phe Ala Asp Asp His Pro
                   85                  90                  95
   Gly Gly Thr Val Ile Ser Thr Tyr Phe Gly Arg Asp Gly Thr Asp Val
               100                 105                 110
   Phe Ala Thr Phe His Pro Pro Ala Ala Trp Lys Gln Leu Asn Asp Tyr
           115                 120                 125
   Tyr Ile Gly Asp Leu Ala Arg Glu Glu Pro Leu Asp Glu Leu Leu Lys
       130                 135                 140
   Asp Tyr Arg Asp Met Arg Ala Glu Phe Val Arg Glu Gly Leu Phe Lys
   145                 150                 155                 160
   Ser Ser Lys Ala Trp Phe Leu Leu Gln Thr Leu Ile Asn Ala Ala Leu
                   165                 170                 175
   Phe Ala Ala Ser Ile Ala Thr Ile Cys Tyr Asp Lys Ser Tyr Trp Ala
               180                 185                 190
   Ile Val Leu Ser Ala Ser Leu Met Gly Leu Phe Val Gln Gln Cys Gly
           195                 200                 205
   Trp Leu Ala His Asp Phe Leu His Gln Gln Val Phe Glu Asn Arg Thr
       210                 215                 220
   Ala Asn Ser Phe Phe Gly Tyr Leu Phe Gly Asn Cys Val Leu Gly Phe
   225                 230                 235                 240
   Ser Val Ser Trp Trp Arg Thr Lys His Asn Ile His His Thr Ala Pro
   245 250 255
   Asn Glu Cys Asp Glu Gln Tyr Thr Pro Leu Asp Glu Asp Ile Asp Thr
   260 265 270
   Leu Pro Ile Ile Ala Trp Ser Lys Glu Ile Leu Ala Thr Val Glu Ser
   275 280 285
   Lys Arg Ile Leu Arg Val Leu Gln Tyr Gln His Tyr Met Ile Leu Pro
   290 295 300
   Leu Leu Phe Met Ala Arg Tyr Ser Trp Thr Phe Gly Ser Leu Leu Phe
   305 310 315 320
   Thr Phe Asn Pro Asp Leu Ser Thr Thr Lys Gly Leu Ile Glu Lys Gly
   325 330 335
   Thr Val Ala Phe His Tyr Ala Trp Phe Ser Trp Ala Ala Phe His Ile
   340 345 350
   Leu Pro Gly Val Ala Lys Pro Leu Ala Trp Met Val Ala Thr Glu Leu
   355 360 365
   Val Ala Gly Leu Leu Leu Gly Phe Val Phe Thr Leu Ser His Asn Gly
   370 375 380
   Lys Glu Val Tyr Asn Glu Ser Lys Asp Phe Val Arg Ala Gln Val Ile
   385 390 395 400
   Thr Thr Arg Asn Thr Lys Arg Gly Trp Phe Asn Asp Trp Phe Thr Gly
   405 410 415
   Gly Leu Asp Thr Gln Ile Glu His His Leu Phe Pro Thr Met Pro Arg
   420 425 430
   His Asn Tyr Pro Lys Ile Ala Pro Gln Val Glu Ala Leu Cys Lys Lys
   435 440 445
   His Gly Leu Glu Tyr Asp Asn Val Ser Val Val Gly Ala Ser Val Ala
   450 455 460
   Val Val Lys Ala Leu Lys Glu Ile Ala Asp Glu Ala Ser Ile Arg Leu
   465 470 475 480
   His Ala His
<210> 5
   <211> 520
<212> DNA
   <213> Ceratodon purpureus
<400> 5
   cattcatcat actgctccga atgagtgcga cgaacagtac acacctctag acgaagacat 60
   tgatactctc cccatcattg cctggagcaa ggaaattttg gccaccgttg agagcaagag 120
   aattttgcga gtgcttcgat atcagcacta catgattctg cctctattgt tcatggcccg 180
   gtacagttgg acttttggaa gtttgctctt cacattcaat cctgatttga gcacgaccaa 240
   gggattgata gagaagggaa cagttgcttt tcactacgcc tggttcagtt gggctgcgtt 300
   ccatattttg ccgggtgtcg ctaagcctct tgcgtggatg gtagcaactg agcttgtggc 360
   cggtttgttg ttgggattcg tgtttacgtt gagtcacaat ggaaaggagg tttacaatga 420
   atcgaaggac ttcgtgagag cccaggttat taccacccgt aacaccaagc gaggctggtt 480
   caacgattgg ttcactgggg gactcgacac ccagattgag                       520
<210> 6
   <211> 173
   <212> PRT
   <213> Ceratodon purpureus
<400> 6
   Ile His His Thr Ala Pro Asn Glu Cys Asp Glu Gln Tyr Thr Pro Leu
   1 5 10 15
   Asp Glu Asp Ile Asp Thr Leu Pro Ile Ile Ala Trp Ser Lys Glu Ile
   20 25 30
   Leu Ala Thr Val Glu Ser Lys Arg Ile Leu Arg Val Leu Gln Tyr Gln
   35 40 45
   His Tyr Met Ile Leu Pro Leu Leu Phe Met Ala Arg Tyr Ser Trp Thr
   50 55 60
   Phe Gly Ser Leu Leu Phe Thr Phe Asn Pro Asp Leu Ser Thr Thr Lys
   65 70 75 80
   Gly Leu Ile Glu Lys Gly Thr Val Ala Phe His Tyr Ala Trp Phe Ser
   85 90 95
   Trp Ala Ala Phe His Ile Leu Pro Gly Val Ala Lys Pro Leu Ala Trp
   100 105 110
   Met Val Ala Thr Glu Leu Val Ala Gly Leu Leu Leu Gly Phe Val Phe
   115 120 125
   Thr Leu Ser His Asn Gly Lys Glu Val Tyr Asn Glu Ser Lys Asp Phe
   130 135 140
   Val Arg Ala Gln Val Ile Thr Thr Arg Asn Thr Lys Arg Gly Trp Phe
   145 150 155 160
   Asn Asp Trp Phe Thr Gly Gly Leu Asp Thr Gln Ile Glu
   165 170
<210> 7
   <211> 514
   <212> DNA
   <213> Ceratodon purpureus
<400> 7
   cctgcatcat gctgctccga atgaatgcga ccaaaagtac acgccgattg atgaggatat 60
   tgatactctc cccatcattg cttggagtaa agatctcttg gccactgttg agagcaagac 120
   catgttgcga gttcttcagt accagcacct attctttttg gttcttttga cgtttgcccg 180
   ggcgagttgg ctattttgga gcgcggcctt cactctcagg cccgagttga cccttggcga 240
   gaagcttttg gagaggggaa cgatggcttt gcactacatt tggtttaata gtgttgcgtt 300
   ttatctgctc cccggatgga aaccagttgt atggatggtg gtcagcgagc tcatgtctgg 360
   tttcctgctg ggatacgtat ttgtactcag tcacaatgga atggaggtgt acaatacgtc 420
   aaaggacttc gtgaatgccc agattgcatc gactcgcgac atcaaagcag gggtgtttaa 480
   tgattggttc accggaggtc tcaacagaca gatt 514
<210> 8
   <211> 172
   <212> PRT
   <213> Ceratodon purpureus
<400> 8
   Leu His His Ala Ala Pro Asn Glu Cys Asp Gln Lys Tyr Thr Pro Ile
   1 5 10 15
   Asp Glu Asp Ile Asp Thr Leu Pro Ile Ile Ala Trp Ser Lys Asp Leu
   20 25 30
   Leu Ala Thr Val Glu Ser Lys Thr Met Leu Arg Val Leu Gln Tyr Gln
   35 40 45
   His Leu Phe Phe Leu Val Leu Leu Thr Phe Ala Arg Ala Ser Trp Leu
   50 55 60
   Phe Trp Ser Ala Ala Phe Thr Leu Arg Pro Glu Leu Thr Leu Gly Glu
   65 70 75 80
   Lys Leu Leu Glu Arg Gly Thr Met Ala Leu His Tyr Ile Trp Phe Asn
   85 90 95
   Ser Val Ala Phe Tyr Leu Leu Pro Gly Trp Lys Pro Val Val Trp Met
   100 105 110
   Val Val Ser Glu Leu Met Ser Gly Phe Leu Leu Gly Tyr Val Phe Val
   115 120 125
   Leu Ser His Asn Gly Met Glu Val Tyr Asn Thr Ser Lys Asp Phe Val
   130 135 140
   Asn Ala Gln Ile Ala Ser Thr Arg Asp Ile Lys Ala Gly Val Phe Asn
   145 150 155 160
   Asp Trp Phe Thr Gly Gly Leu Asn Arg Gln Ile Glu
   165 170
<210> 9
   <211> 535
   <212> DNA
   <213> Ceratodon purpureus
<400> 9
   tgctcatcac atcgcctgta atagtataga atatgatcca gacctacagt acatccccct 60
   ttttgcagtg acatcaaagc tcttctctaa cctctactcc tacttctatg aaagggttat 120
   gccattcgat ggcgtagcac gctctctgat tgcctaccag cactggacgt tttatccaat 180
   aatggctgtt gctcgggtga acctctttgc ccaatccctt ctagtactga cctcgaagaa 240
   gcatgtgcca gacaggtggc ttgagctcgg tgctatcggt ttcttctacc tgtggttctt 300
   caccctcttg tcgtacctgc ccactgcacc ggagaggctt gctttcgtcc ttgtcagttt 360
   tgcagtgaca gggatccagc atgtacagtt ttgcctgaac cacttctcat cgccggttta 420
   tctaggacag ccgaagagca aggcttgggt tgaatctcaa gcacggggca ctctcaatct 480
   ctctacaccg gcttacatgg attggtttca cgggggtctt cagttccaga tcgag 535
<210> 10
   <211> 178
   <212> PRT
   <213> Ceratodon purpureus
<400> 10
   Ala His His Ile Ala Cys Asn Ser Ile Glu Tyr Asp Pro Asp Leu Gln
   1               5                   10                  15
   Tyr Ile Pro Leu Phe Ala Val Thr Ser Lys Leu Phe Ser Asn Leu Tyr
               20                  25                  30
   Ser Tyr Phe Tyr Glu Arg Val Met Pro Phe Asp Gly Val Ala Arg Ser
           35                  40                  45
   Leu Ile Ala Tyr Gln His Trp Thr Phe Tyr Pro Ile Met Ala Val Ala
       50                  55                  60
   Arg Val Asn Leu Phe Ala Gln Ser Leu Leu Val Leu Thr Ser Lys Lys
   65                  70                  75                  80
   His Val Pro Asp Arg Trp Leu Glu Leu Gly Ala Ile Gly Phe Phe Tyr
                   85                  90                  95
   Leu Trp Phe Phe Thr Leu Leu Ser Tyr Leu Pro Thr Ala Pro Glu Arg
               100                 105                 110
   Leu Ala Phe Val Leu Val Ser Phe Ala Val Thr Gly Ile Gln His Val
           115                 120                 125
   Gln Phe Cys Leu Asn His Phe Ser Ser Pro Val Tyr Leu Gly Gln Pro
       130                 135                 140
   Lys Ser Lys Ala Trp Val Glu Ser Gln Ala Arg Gly Thr Leu Asn Leu
   145                 150                 155                 160
   Ser Thr Pro Ala Tyr Met Asp Trp Phe His Gly Gly Leu Gln Phe Gln
                   165                 170                 175
   Ile Glu
<210> 11
   <211> 2160
   <212> DNA
   <213> Ceratodon purpureus
<220>
   <221> CDS
   <222> (159)..(1721)
<400> 11
   cggaggtctc ttgtcgttct tggagtctgt gtcgagcttg gaatgcggta ggcgcggccg 60
   tttcgtggtt ttggcgttgg cattgcgcga gggcggacag tgggagtgcg ggaggtctgt 120
   ttgtgcatga cgaggtggtt gtaatcttcg ccggcaga atg gtg tcc cag ggc ggc 176
                                            Met Val Ser Gln Gly Gly
                                             1               5
   ggt ctc tcg cag ggt tcc att gaa gaa aac att gac gtt gag cac ttg   224
   Gly Leu Ser Gln Gly Ser Ile Glu Glu Asn Ile Asp Val Glu His Leu
               10                  15                  20
   gca acg atg ccc ctc gtc agt gac ttc cta aat gtc ctg gga acg act   272
   Ala Thr Met Pro Leu Val Ser Asp Phe Leu Asn Val Leu Gly Thr Thr
           25                  30                  35
   ttg ggc cag tgg agt ctt tcc act aca ttc gct ttc aag agg ctc acg   320
   Leu Gly Gln Trp Ser Leu Ser Thr Thr Phe Ala Phe Lys Arg Leu Thr
       40                  45                  50
   act aag aaa cac agt tcg gac atc tcg gtg gag gca caa aaa gaa tcg   368
   Thr Lys Lys His Ser Ser Asp Ile Ser Val Glu Ala Gln Lys Glu Ser
    55                  60                  65                  70
   gtt gcg cgg ggg cca gtt gag aat att tct caa tcg gtt gcg cag ccc   416
   Val Ala Arg Gly Pro Val Glu Asn Ile Ser Gln Ser Val Ala Gln Pro
                   75                  80                  85
   atc agg cgg agg tgg gtg cag gat aaa aag ccg gtt act tac agc ctg   464
   Ile Arg Arg Arg Trp Val Gln Asp Lys Lys Pro Val Thr Tyr Ser Leu
               90                  95                  100
   aag gat gta gct tcg cac gat atg ccc cag gac tgc tgg att ata atc   512
   Lys Asp Val Ala Ser His Asp Met Pro Gln Asp Cys Trp Ile Ile Ile
           105                 110                 115
   aaa gag aag gtg tat gat gtg agc acc ttc gct gag cag cac cct gga   560
   Lys Glu Lys Val Tyr Asp Val Ser Thr Phe Ala Glu Gln His Pro Gly
       120                 125                 130
   ggc acg gtt atc aac acc tac ttc gga cga gac gcc aca gat gtt ttc   608
   Gly Thr Val Ile Asn Thr Tyr Phe Gly Arg Asp Ala Thr Asp Val Phe
   135                 140                 145                 150
   tct act ttc cac gca tcc acc tca tgg aag att ctt cag aat ttc tac   656
   Ser Thr Phe His Ala Ser Thr Ser Trp Lys Ile Leu Gln Asn Phe Tyr
                   155                 160                 165
   atc ggg aac ctt gtt agg gag gag ccg act ttg gag ctg ctg aag gag   704
   Ile Gly Asn Leu Val Arg Glu Glu Pro Thr Leu Glu Leu Leu Lys Glu
               170                 175                 180
   tac aga gag ttg aga gcc ctt ttc ttg aga gaa cag ctt ttc aag agt     752
   Tyr Arg Glu Leu Arg Ala Leu Phe Leu Arg Glu Gln Leu Phe Lys Ser
           185                 190                 195
   tcc aaa tcc tac tac ctt ttc aag act ctc ata aat gtt tcc att gtt     800
   Ser Lys Ser Tyr Tyr Leu Phe Lys Thr Leu Ile Asn Val Ser Ile Val
       200                 205                 210
   gcc aca agc att gcg ata atc agt ctg tac aag tct tac cgg gcg gtt     848
   Ala Thr Ser Ile Ala Ile Ile Ser Leu Tyr Lys Ser Tyr Arg Ala Val
   215                 220                 225                 230
   ctg tta tca gcc agt ttg atg ggc ttg ttt att caa cag tgc gga tgg     896
   Leu Leu Ser Ala Ser Leu Met Gly Leu Phe Ile Gln Gln Cys Gly Trp
                   235                 240                 245
   ttg tct cac gat ttt cta cac cat cag gta ttt gag aca cgc tgg ctc     944
   Leu Ser His Asp Phe Leu His His Gln Val Phe Glu Thr Arg Trp Leu
               250                 255                 260
   aat gac gtt gtt ggc tat gtg gtc ggc aac gtt gtt ctg gga ttc agt     992
   Asn Asp Val Val Gly Tyr Val Val Gly Asn Val Val Leu Gly Phe Ser
           265                 270                 275
   gtc tcg tgg tgg aag acc aag cac aac ctg cat cat gct gct ccg aat     1040
   Val Ser Trp Trp Lys Thr Lys His Asn Leu His His Ala Ala Pro Asn
       280                 285                 290
   gaa tgc gac caa aag tac aca ccg att gat gag gat att gat act ctc     1088
   Glu Cys Asp Gln Lys Tyr Thr Pro Ile Asp Glu Asp Ile Asp Thr Leu
   295                 300                 305                 310
   ccc atc att gct tgg agt aaa gat ctc ttg gcc act gtt gag agc aag     1136
   Pro Ile Ile Ala Trp Ser Lys Asp Leu Leu Ala Thr Val Glu Ser Lys
                   315                 320                 325
   acc atg ttg cga gtt ctt cag tac cag cac cta ttc ttt ttg gtt ctt     1184
   Thr Met Leu Arg Val Leu Gln Tyr Gln His Leu Phe Phe Leu Val Leu
               330                 335                 340
   ttg acg ttt gcc cgg gcg agt tgg cta ttt tgg agc gcg gcc ttc act     1232
   Leu Thr Phe Ala Arg Ala Ser Trp Leu Phe Trp Ser Ala Ala Phe Thr
           345                 350                 355
   ctc agg ccc gag ttg acc ctt ggc gag aag ctt ttg gag agg gga acg     1280
   Leu Arg Pro Glu Leu Thr Leu Gly Glu Lys Leu Leu Glu Arg Gly Thr
       360                 365                 370
   atg gct ttg cac tac att tgg ttt aat agt gtt gcg ttt tat ctg ctc    1328
   Met Ala Leu His Tyr Ile Trp Phe Asn Ser Val Ala Phe Tyr Leu Leu
   375                 380                 385                 390
   ccc gga tgg aaa cca gtt gta tgg atg gtg gtc agc gag ctc atg tct    1376
   Pro Gly Trp Lys Pro Val Val Trp Met Val Val Ser Glu Leu Met Ser
                   395                 400                 405
   ggt ttc ctg ctg gga tac gta ttt gta ctc agt cac aat gga atg gag    1424
   Gly Phe Leu Leu Gly Tyr Val Phe Val Leu Ser His Asn Gly Met Glu
               410                 415                 420
   gtg tac aat acg tca aag gac ttc gtg aat gcc cag att gca tcg act    1472
   Val Tyr Asn Thr Ser Lys Asp Phe Val Asn Ala Gln Ile Ala Ser Thr
           425                 430                 435
   cgc gac atc aaa gca ggg gtg ttt aat gat tgg ttc acc gga ggt ctc    1520
   Arg Asp Ile Lys Ala Gly Val Phe Asn Asp Trp Phe Thr Gly Gly Leu
       440                 445                 450
   aac aga cag att gag cat cat cta ttt cca acg atg ccc agg cac aac    1568
   Asn Arg Gln Ile Glu His His Leu Phe Pro Thr Met Pro Arg His Asn
   455                 460                 465                 470
   ctt aat aaa att tct cct cac gtg gag act ttg tgc aag aag cat gga    1616
   Leu Asn Lys Ile Ser Pro His Val Glu Thr Leu Cys Lys Lys His Gly
                   475                 480                 485
   ctg gtc tac gaa gac gtg agc atg gct tcg ggc act tac cgg gtt ttg    1664
   Leu Val Tyr Glu Asp Val Ser Met Ala Ser Gly Thr Tyr Arg Val Leu
               490                 495                 500
   aaa aca ctt aag gac gtt gcc gat gct gct tca cac cag cag ctt gct    1712
   Lys Thr Leu Lys Asp Val Ala Asp Ala Ala Ser His Gln Gln Leu Ala
           505                 510                 515
   gcg agt tga ggcatcgcag cactcgtcga aacatttttg tctgttatag            1761
   Ala Ser
       520
   tgttcatatg tgatcgaggg gaaaaggtcc catgctctga tctattcttc tgtagccaat 1821
   atttttcaat tgaaaggagg ttcctcactt atcttccatc tatcgttgca catcctgcat 1881
   cagagttagc gttggagtaa tgttaagcac ttgtagatta tgcccaccat tgccacattt 1941
   ctgttcggtt acaatcgttt gattccatgc tatcctccgt gttcatctcg ttgttataag 2001
   caagcttgaa aaaacatgct acgagattgg cagacgttgt cttggcagct gtagaggttg 2061
   gttccattca ttgtgtagta cagaactctc tcgtccctgt ttctctacat tacttgttac 2121
   atagtgactt tcattcacag caaaaaaaaa aaaaaaaaa 2160
<210> 12
   <211> 520
   <212> PRT
   <213> Ceratodon purpureus
<400> 12
   Met Val Ser Gln Gly Gly Gly Leu Ser Gln Gly Ser Ile Glu Glu Asn
    1               5                  10                  15
   Ile Asp Val Glu His Leu Ala Thr Met Pro Leu Val Ser Asp Phe Leu
               20                  25                  30
   Asn Val Leu Gly Thr Thr Leu Gly Gln Trp Ser Leu Ser Thr Thr Phe
           35                  40                  45
   Ala Phe Lys Arg Leu Thr Thr Lys Lys His Ser Ser Asp Ile Ser Val
       50                  55                  60
   Glu Ala Gln Lys Glu Ser Val Ala Arg Gly Pro Val Glu Asn Ile Ser
   65                  70                  75                  80
   Gln Ser Val Ala Gln Pro Ile Arg Arg Arg Trp Val Gln Asp Lys Lys
                   85                  90                  95
   Pro Val Thr Tyr Ser Leu Lys Asp Val Ala Ser His Asp Met Pro Gln
               100                 105                 110
   Asp Cys Trp Ile Ile Ile Lys Glu Lys Val Tyr Asp Val Ser Thr Phe
           115                 120                 125
   Ala Glu Gln His Pro Gly Gly Thr Val Ile Asn Thr Tyr Phe Gly Arg
       130                 135                 140
   Asp Ala Thr Asp Val Phe Ser Thr Phe His Ala Ser Thr Ser Trp Lys
   145                 150                 155                 160
   Ile Leu Gln Asn Phe Tyr Ile Gly Asn Leu Val Arg Glu Glu Pro Thr
                   165                 170                 175
   Leu Glu Leu Leu Lys Glu Tyr Arg Glu Leu Arg Ala Leu Phe Leu Arg
               180                 185                 190
   Glu Gln Leu Phe Lys Ser Ser Lys Ser Tyr Tyr Leu Phe Lys Thr Leu
           195                 200                 205
   Ile Asn Val Ser Ile Val Ala Thr Ser Ile Ala Ile Ile Ser Leu Tyr
       210                 215                 220
   Lys Ser Tyr Arg Ala Val Leu Leu Ser Ala Ser Leu Met Gly Leu Phe
   225 230 235 240
   Ile Gln Gln Cys Gly Trp Leu Ser His Asp Phe Leu His His Gln Val
   245 250 255
   Phe Glu Thr Arg Trp Leu Asn Asp Val Val Gly Tyr Val Val Gly Asn
   260 265 270
   Val Val Leu Gly Phe Ser Val Ser Trp Trp Lys Thr Lys His Asn Leu
   275 280 285
   His His Ala Ala Pro Asn Glu Cys Asp Gln Lys Tyr Thr Pro Ile Asp
   290 295 300
   Glu Asp Ile Asp Thr Leu Pro Ile Ile Ala Trp Ser Lys Asp Leu Leu
   305 310 315 320
   Ala Thr Val Glu Ser Lys Thr Met Leu Arg Val Leu Gln Tyr Gln His
   325 330 335
   Leu Phe Phe Leu Val Leu Leu Thr Phe Ala Arg Ala Ser Trp Leu Phe
   340 345 350
   Trp Ser Ala Ala Phe Thr Leu Arg Pro Glu Leu Thr Leu Gly Glu Lys
   355 360 365
   Leu Leu Glu Arg Gly Thr Met Ala Leu His Tyr Ile Trp Phe Asn Ser
   370 375 380
   Val Ala Phe Tyr Leu Leu Pro Gly Trp Lys Pro Val Val Trp Met Val
   385 390 395 400
   Val Ser Glu Leu Met Ser Gly Phe Leu Leu Gly Tyr Val Phe Val Leu
   405 410 415
   Ser His Asn Gly Met Glu Val Tyr Asn Thr Ser Lys Asp Phe Val Asn
   420 425 430
   Ala Gln Ile Ala Ser Thr Arg Asp Ile Lys Ala Gly Val Phe Asn Asp
   435 440 445
   Trp Phe Thr Gly Gly Leu Asn Arg Gln Ile Glu His His Leu Phe Pro
   450 455 460
   Thr Met Pro Arg His Asn Leu Asn Lys Ile Ser Pro His Val Glu Thr
   465 470 475 480
   Leu Cys Lys Lys His Gly Leu Val Tyr Glu Asp Val Ser Met Ala Ser
   485 490 495
   Gly Thr Tyr Arg Val Leu Lys Thr Leu Lys Asp Val Ala Asp Ala Ala
               500                 505                 510
   Ser His Gln Gln Leu Ala Ala Ser
           515                 520